# EUROPÄISCHE PATENTSCHRIFT

(11) **EP 1 109 785 B1**
(45) Veröffentlichungstag und Bekanntmachung des Hinweises auf die Patenterteilung: **02.01.2003**
(21) Anmeldenummer: 99927711.4
(22) Anmeldetag: 22.04.1999
(51) Int. Cl.: C07D 209/14, A61K 31/40, A61K 31/38, A61K 31/47, C07D 333/56, C07D 409/06, C07D 405/06, C07D 487/14, C07D 491/14, C07D 403/14, C07H 19/04, C07F 7/10, C07D 401/14, C07D 487/22, C07D 471/22

(54) **INDOLDERIVATE UND DEREN VERWENDUNG ZUR BEHANDLUNG VON MALIGNEN UND ANDEREN, AUF PATHOLOGISCHEN ZELLPROLIFERATIONEN BERUHENDEN ERKRANKUNGEN**
INDOLE DERIVATIVES AND THEIR USE IN THE TREATMENT OF MALIGNANT AND OTHER DISEASES CAUSED BY PATHOLOGICAL CELL PROLIFERATION
DERIVES INDOLIQUES ET LEUR UTILISATION POUR LE TRAITEMENT DE MALADIES MALIGNES ET AUTRES INDUITES PAR DES PROLIFERATIONS CELLULAIRES PATHOLOGIQUES

(30) Priorität: 04.05.1998 DE 19819835; 25.08.1998 DE 19838506
(43) Veröffentlichungstag der Anmeldung: 27.06.2001
(73) Patentinhaber: Zentaris AG, 60314 Frankfurt/Main (DE)
(72) Erfinder: MAHBOOBI, Siavosh, D-93051 Regensburg (DE); DE WALL, Sabine, D-26655 Westerstede (DE); PONGRATZ, Herwig, D-93053 Regensburg (DE); POPP, Alfred, D-84489 Burghausen (DE); HUFSKY, Harald, D-85080 Gaimersheim (DE); BÖHMER, Frank-D., D-07778 Dorndorf (DE); TELLER, Steffen, D-07743 Jena (DE); UECKER, Andrea, 07745 Jena (DE); BECKERS, Thomas, D-60596 Frankfurt (DE)
(86) Internationale Anmeldenummer: DE9901214
(87) Internationale Veröffentlichungsnummer: WO99057117

(56) Entgegenhaltungen:
- EP-A- 0 778 274
- WO-A-94/24117
- WO-A-95/17182
- CHEMICAL ABSTRACTS, vol. 126, no. 10, 1997 Columbus, Ohio, US; abstract no. 132951r, A. KONDO ET AL.: "Bisazo compound, its intermediate and their manufacture and electrophotographic photoreceptor with high-stability and durability" Seite 711; XP002120349 & JP 08 295688 A (SHARP KK) 12. November 1996 (1996-11-12)
- CHEMICAL ABSTRACTS, vol. 115, no. 15, 1991 Columbus, Ohio, US; abstract no. 158875s, C.F. NUTAITIS ET AL.: "Reduction of heterocyclic alcohols with sodium borohydridr-trifluoroacetic acid. preparation of bis-heterocyclic methanes" Seite 911; XP002120350 & ORG. PREP. PROCED. INT., Bd. 23, Nr. 4, 1991, Seiten 403-411,
- E. FISCHER ET AL: "Synthesis of new sulfur heteroaromatics isoelectronic with dibenzo[g,p]chrysene by photocylization of thienyl- and phenyl-sunstituted ethenes" JOURNAL OF ORGANIC CHEMISTRY., Bd. 61, Nr. 20, 1996, Seiten 6997-7005, XP002120346 AMERICAN CHEMICAL SOCIETY. EASTON., US ISSN: 0022-3263
- CHEMICAL ABSTRACTS, vol. 107, no. 5, 1987 Columbus, Ohio, US; abstract no. 39594z, J.M. GONZALEZ: "Process for the preparaton of 2,2'-dibenzofurylic compounds" Seite 678; XP002120351 & ES 538 693 A (MENARINI) 16. März 1986 (1986-03-16)
- CHEMICAL ABSTRACTS, vol. 105, no. 21, 1986 Columbus, Ohio, US; abstract no. 190793t, M. NAGAHARA ET AL.: "Synthesis and antiviral activity of bis(hydroxy-2-benzofuranyl)ketone derivative" Seite 702; XP002120352 & YAKUGAKU ZASSHI, Bd. 105, Nr. 9, 1985, Seiten 840-44,
- O. DANN ET AL.: "Trypanocide Diamidine mit vier Ringen in einem oder zwei Ringsystemen" JUSTUS LIEBIGS ANNALEN DER CHEMIE., 1973, Seiten 1112-1140, XP002120347 VERLAG CHEMIE GMBH. WEINHEIM., DE ISSN: 0075-4617
- M. AHMED ET AL.: "The direct Bradsher reaction. Part I. Synthesis of thiophen analogues of linear polycyclic hydrocarbons" JOURNAL OF THE CHEMICAL SOCIETY, PERKIN TRANSACTIONS 1., Nr. 10, 1973, Seiten 1099-103, XP002120348 CHEMICAL SOCIETY. LETCHWORTH., GB ISSN: 0300-922X

## Beschreibung

Die Erfindung betrifft Tyrosinkinase-Inhibitoren vom Typ der Bis-indolyl-Verbindungen, diese enthaltende Arzneimittel und deren Verwendung zur Behandlung von malignen und anderen, auf pathologischen Zellproliferationen beruhenden Erkrankungen.

Die Aktivierung von Tyrosin-spezifischen Proteinkinasen ist ein Schlüsselereignis für die Stimulation der Teilung tierischer Zellen. Normalerweise erfolgt diese Stimulation durch exogene Faktoren, z.B. Wachstumsfaktoren, wenn die Proliferation eines bestimmten Zelltyps für die Gesamtfunktion eines Gewebes oder Organs erforderlich ist. In Tumoren ist die Zellpmliferation ebenfalls mit der Aktivität von Tyrosinkinasen verknüpft. Häufig liegt in Tumorzellen jedoch eine aberrante Aktivität von Kinasen vor, welche durch Überexpression, konstitutiv aktive Kinase-Mutanten oder ektopische Aktivität von Wachstumsfaktoren verursacht ist. Der PDGF-Rezeptor ist einer der Wachstumsfaktoren mit Relevanz für menschliche Tumoren. PDGF stellt eines der Hauptmitogene im Serum dar und liegt in hohen Konzentrationen in Blutplättchen vor. Seine wichtigste Funktion im adulten Organismus ist die Wundheilung. Eine unerwünschte Aktivität des PDGF-Rezeptors ist an der Proliferation von verschiedenen Tumoren, z:B. Gliomen, Glioblastomen, Sarkomen, Mammakarzinomen, Ovarialkarzinomen und Kolonkarzinomen, beteiligt. Eine aberrante Aktivierung des PDGF/PDGF-Rezeptor-Systems nimmt auch eine Schlüsselstellung für pathologische Hyperproliferationen mesenchymaler Zellen im Kontext von Arteriosklerose, Restenose nach Ballon-Angioplasie, Arthritis und fibrotischen Erkrankungen ein.

Einige Wachstumsfaktor-Rezeptortyrosinkinasen, deren Tyrosinkinase-Domänen hohe Sequenzhomologie zur Tyrosinkinase-Domäne der PDGF-Rezeptoren aufweisen, sind ebenfalls von Bedeutung für das Tumorgeschehen und pathologische Hyperproliferationen. Dazu zählen die Rezeptoren für den Vaskulären Endothelzellwachstumsfaktor (VEGF) KDR/Flk-1 und Flt-1 mit großer Bedeutung für die Tumorvaskularisation, Kit/SCF-Rezeptor, für den konstitutiv aktive Versionen in Karzinomen beobachtet wurden und Flk-2/Flt-3, ein an der Proliferation von Leukämiezellen verschiedener Erkrankungsformen beteiligter Rezeptor. Es kann erwartet werden, daß weitere Mitglieder dieser Kinase-Familie mit Relevanz für pathologische Proliferationen identifiziert werden. Zu den Wirkungen der Liganden dieser Rezeptoren zählt neben der mitogenen Stimulation häufig auch die Stimulation der Zellmigration, anti-apoptotische Wirkungen und Effekte auf Membran-Transportsysteme für lonen, Wasser und chemische Verbindungen. Unkontrollierte Effekte dieses Typs sind in verschiedenem Ausmaß ebenfalls am pathologischen Geschehen in Tumoren und anderen Erkrankungen beteiligt.

Unter verschiedenen Möglichkeiten, das Signal von Rezeptor-Tyrosinkinasen abzuschalten, ist die spezifische direkte Hemmung der Aktivität der Kinase am aussichtsreichsten.

Aus dem Stand der Technik sind Veröffentlichungen bekannt, die Dibenzofuryl- und Di-benzothienyl-Verbindungen für unterschiedliche Indikationen beschreiben. WO 95/17182 beschreibt Bisindol-Verbindungen, die selektiv die Proteinkinase C inhibieren und Wirkungen bei Tumorerkrankungen, Entzündungsprozessen und Erkrankungen des zentralen Nervemsystems zeigen.

Die Erfindung ist daher darauf gerichtet, Verbindungen zu schaffen, die als Inhibitoren von Tyrosinkinasen, insbesondere der PDGF-Rezeptor-Tyrosinkinasen, sowie weiterer, verwandter Tyrosinkinasen wie KDR/FIk-1, Kit/SCF-Rezeptor und FLK/Flt-3 geeignet sind. Diese Aufgabe wird durch die erfindungsgemäßen Verbindungen der allgemeinen Formel I gelöst: 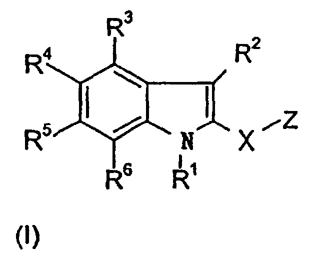 worin Z eine Gruppe mit der allgemeinen Formel (II) ist, 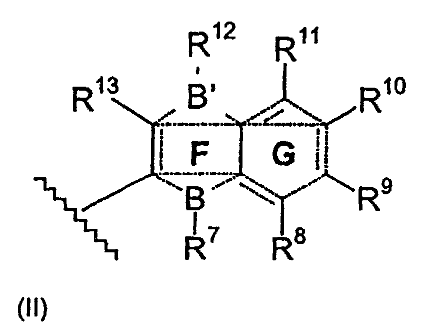 worin B, B' ein Kohlenstoff-, Stickstoff-, Sauerstoff- oder Schwefelatom sein kann und die Ringsysteme F und G unabhängig voneinander sowohl gesättigte als auch ungesättigte 5- und 6-Ringe sein können,
X eine Gruppe mit der allgemeinen Formel III oder IV darstellt,

―(CH₂)ₗ―[CR¹⁴R¹⁵]ₘ―(CH₂)ₙ (III)

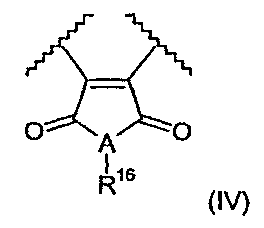

worin A ein N- O-, S-Atom sein kann, I und n die Zahlen von 0 bis 6, m die Zahlen 1 und 2 einnehmen können, sowie R¹⁴ und R¹⁵ entweder zusammen ein Sauerstoffatom bilden oder R¹⁴ eine Hydroxylgruppe und R¹⁵ ein Wasserstoffatom bedeuten oder R¹⁴ und R¹⁵ Wasserstoffatome bedeuten und wobei R¹⁶ ein Wasserstoffatom, ein Alkyl- oder Arylrest, halogen-, amino, oder azidosubstituierter Alkyl- oder Arylrest, ein Alkyloxymethyl oder substituierter Alkyloxymethylrest bedeutet,
R² und R¹³ zusammen eine Verknüpfung mit der allgemeinen Formel V oder VI bilden 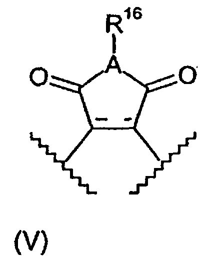 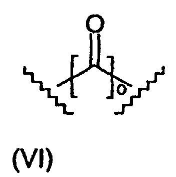 wobei die gestrichelte Bindung eine Doppel- oder Einfachbindung bedeutet, A und R¹⁶ die selbe Bedeutung wie oben besitzen und o die Zahlen 1 und 2 annehmen kann,
R² und R¹³ gleiche oder verschieden Reste der allgemeinen Formel VII oder Wasserstoffatome bedeuten, 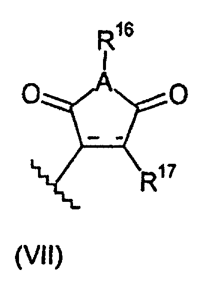 wobei die gestrichelte Bindung eine Doppel- oder Einfachbindung bedeutet, A und R¹⁶ die selbe Bedeutung wie oben besitzen und R¹⁷ ein Halogenatom oder eine n Rest der allgemeinen Formeln VIII bedeutet, 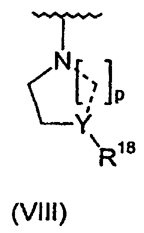 so daß p = 0, 1 oder 2 sein kann (wenn p = 0 dann handelt es sich um ein acyclisches primäres Amin und Y trägt ein zusätzliches Wasserstoffatom), Y ein Kohlenstoff, Sauerstoff- oder Stickstoffatom sein kann und wenn Y ein Kohlenstoff oder Stickstoffatom ist R¹⁸ ein Wasserstoffatom oder einen Alkyl- oder Arylrest, substituierten Alkyl- oder Arylrest, gesättigten oder ungesättigten Heterozyklus, Alkoxycarbonylrest, Aminocarbonylmethylrest, substituierten Aminocarbonylmethylrest bedeutet,
R² und R¹³ zusammen eine Verknüpfung mit der allgemeinen Formel IX oder X bilden 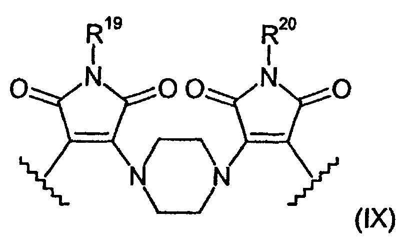 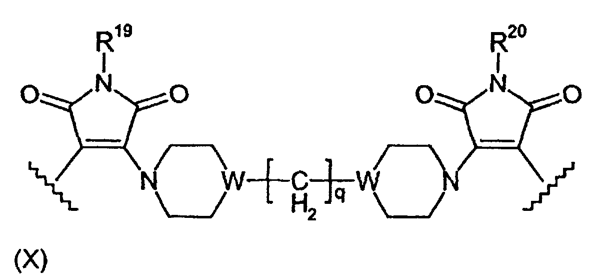 wobei W entweder ein Kohlenstoff- oder ein Stickstoffatom darstellt, q eine Zahl zwischen 0 und 6 einnehmen kann und R¹⁹ und R²⁰ Wasserstoffatome, Alkyl- oder substituierte Alkylreste bedeuten kann,
worin R¹, R⁷ und R¹² gleich oder verschieden sind und Wasserstoffatome, Alkyl- und Aminoalkylreste, Phenylsulphonylreste, Alkylsilylmethoxymethylreste, einen Zucker oder substituierter Zucker bedeuten,
wobei R³, R⁴, R⁵, R⁶, R⁸, R⁹, R¹⁰ und R¹¹ gleich oder verschieden sind und jeweils ein Wasserstoffatom, eine alkoxy-, amino-, halogen-, cycloalkyl-, cycloheteroalkyl-, aryl- oder heteroarylsubstituierte Alkyl-, Alkoxy, Alkoxymethylgruppe, Nitrogruppe, ein Halogenatom oder eine O-Alkoxygruppe der allgemeinen Formel -O-(C=O)-R²¹ darstellt, wobei R²¹ eine alkoxy-, amino-, halogen-, cycloalkyl-, cycloheteroalkyl-, aryl- oder heteroarylsubstituierte Alkyl-, Alkoxy, Alkoxymethylgruppe bedeutet.

Bevorzugt sind erfindungsgemäße Verbindungen mit der obigen allgemeinen Formel I, worin Z eine Gruppe mit der allgemeinen Formel II und X eine Gruppe mit der allgemeinen Formel III darstellt, R² und R¹³ Wasserstoffatome bedeuten, B ein Stickstoff-, Sauerstoff- oder Schwefelatom bedeuten sowie R³, R⁴, R⁵, R⁶, R⁷, R⁸, R⁹, R¹⁰, R¹¹, R¹², R¹⁴ und R¹⁵ dieselbe Bedeutung wie oben haben, wobei diese Verbindungen der folgenden Formel XI entsprechen:

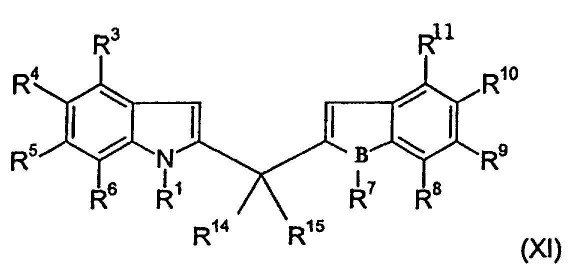

Besonders bevorzugt sind Verbindungen der Formel XI, worin R¹⁴ und R¹⁵ zusammen ein Sauerstoffatom bilden.

Außerdem bevorzugt sind erfindungsgemäße Verbindungen mit den obigen allgemeinen Formeln I worin worin Z eine Gruppe mit der allgemeinen Formel II und X eine Gruppe mit der allgemeinen Formel III darstellt, R² und R¹³ Wasserstoffatome, A und B ein Stickstoffatom bedeuten sowie R¹, R³, R⁴, R⁵, R⁶, R⁷, R⁸, R⁹, R¹⁰, R¹¹, R¹² und R¹⁶ die selbe Bedeutung wie oben haben, wobei diese Verbindungen der folgenden Formel XII entsprechen:

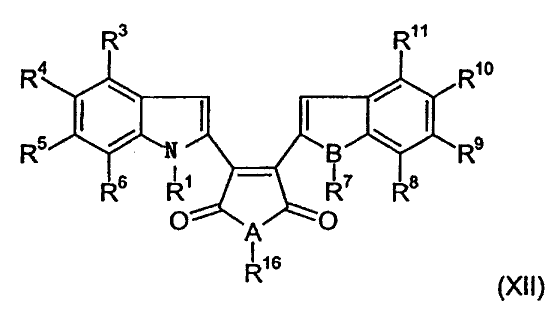

Außerdem bevorzugt sind erfindungsgemäße Verbindungen mit den unten stehenden allgemeinen Formeln XIII und XIV 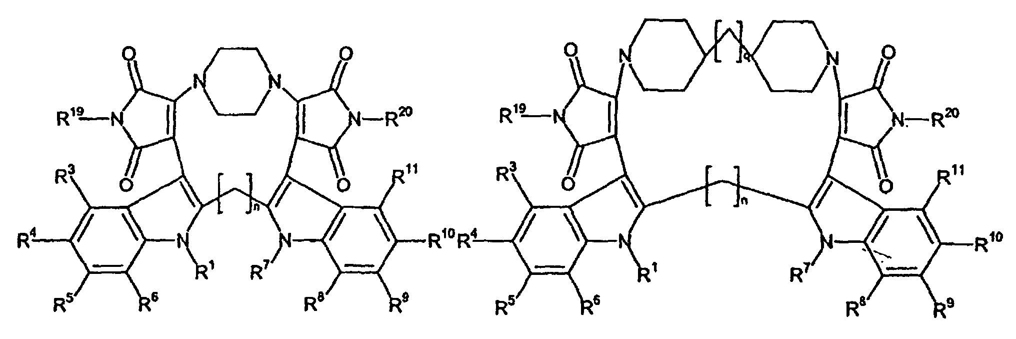 worin n die Zahlen 3, 4, 5, 8, 12, q die Zahlen 0, 1, 2, 3, 5, 6 bedeutet R¹⁹, R²⁰ Wasserstoffatome oder Alkylgruppen bedeuten und R¹, R³, R⁴, R⁵, R⁶, R⁷, R⁸, R⁹, R¹⁰, R¹¹ und R¹⁶ gleich oder verschieden sind und die selbe Bedeutung wie oben besitzen.

Außerdem bevorzugt sind erfindungsgemäße Verbindungen mit der folgenden allgemeinen Formel XV 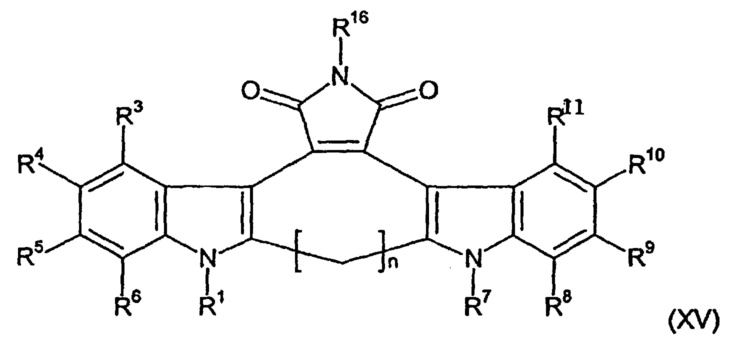 worin n die Zahlen 1, 2, 3 bedeutet, R¹⁶ ein Wasserstoffatom oder eine Alkylgruppe bedeutet und R¹, R³, R⁴, R⁵, R⁶, R⁷, R⁸, R⁹, R¹⁰, R¹¹ und R¹⁶ gleich oder verschieden sind und die selbe Bedeutung wie oben besitzen.

Die Verbindungen der Formel XI sind nach einem der beiden folgenden Schemata herstellbar: 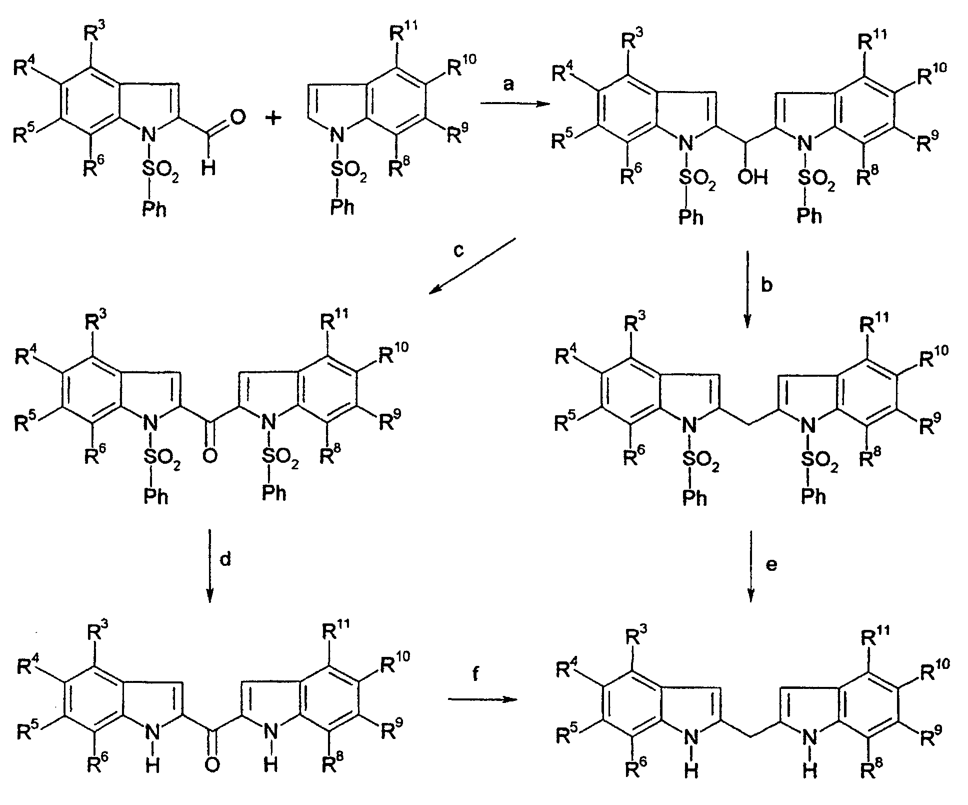 a) LDA/ THF, b) HSiPh₃/THF, c) PDC/ DMF, d) 10% NaOH/ EtOH, e) K₂CO₃/MeOH, f) N₂H₄/ 2-(2-hydroxyethyloxy)-1-ethanol

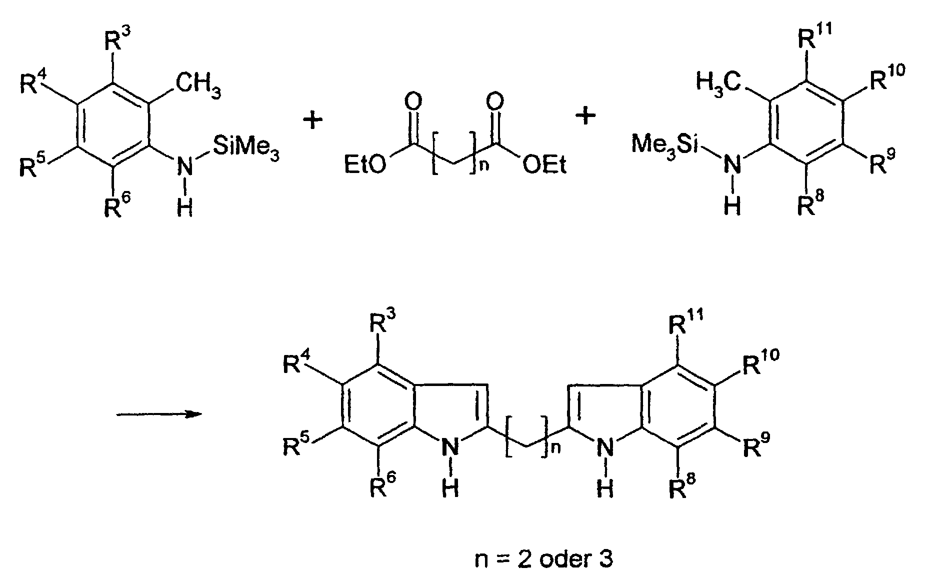

Zur Herstellung der erfindungsgemäßen Verbindungen, bei denen R² und R¹³ einen Rest mit der obigen allgemeinen Formel VII bedeuten oder zusammen eine Verknüpfung mit der allgemeinen Formel V, IX oder X bilden, setzt man zunächst ein 2,2'-Bis-1*H*-Indolylalkan oder ein Derivat desselben mit der allgemeinen Formel Xl 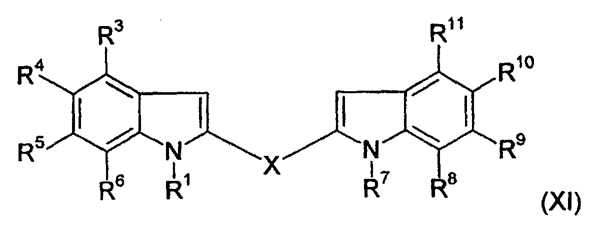 in der X, R¹, R³, R⁴, R⁵, R⁶, R⁷, R⁸, R⁹, R¹⁰ und R¹¹ die gleiche Bedeutung wie oben besitzen, mit Dibrommaleinimid um.

Erfindungsgemäße Verbindungen, bei denen R² und R¹³ zusammen eine Verknüpfung mit der allgemeinen Formel VII bilden, setzt man anschließend mit einem primären oder sekundären Amin mit der folgenden allgemeinen Formel XVI oder XVII oder Piperazin um 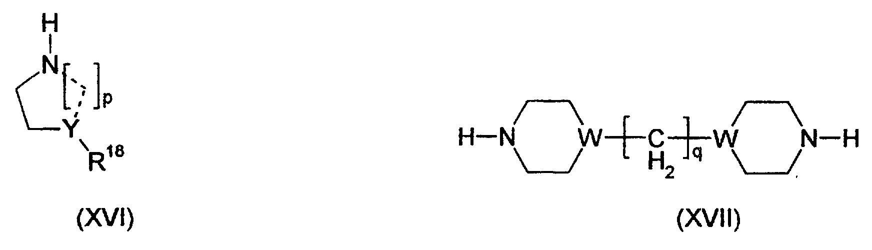 worin p, q, R¹⁷ und W die selbe Bedeutung wie oben besitzen.

Die folgenden Beispiele erläutern die Erfindung, ohne sie zu beschränken.

### Beispiel 1

### Bis(N-phenylsulfonylindol-2-yl)-1-methanol

Bei - 78 °C bereitet man Lithiumdiisopropylamid aus 30.40 ml (216.3 mmol) Diisopropylamin und 125.3 ml (200.5 mmol) n-BuLi (1.6 M in Hexan) in 200 ml absol. THF. Die Lösung wird 10 min bei - 78 °C und anschließend 30 min bei 0 °C gerührt, bevor bei 0 °C 49.13 g (190.9 mmol) 1-Phenylsulfonylindol in 300 ml absol. THF innerhalb von 10 min zugetropft werden. Die Reaktionslösung wird weitere 30 min bei 0 °C gerührt. Nach erneutem Abkühlen auf - 78 °C werden 60.00 g (210.3 mmol) Phenylsulfonyl-2-carbaldehyd in 200 ml absol. THF zugetropft und über Nacht auf Raumtemp. erwärmen lassen. Die Mischung wird auf 1 proz. HCI gegossen und die org. Phase mit nach Zugabe von Ether abgetrennt. Die wäßr. Phase wird mit Ether extrahiert, die vereinigten org. Phasen werden nacheinander mit NaHCO₃ und ges. NaCI-Lösung gewaschen und über Na₂SO₄ getrocknet. Das Lösungsmittel wird i. Vak. abgezogen und das Rohprodukt sc (SiO₂; CH₂Cl₂) gereinigt: farblose Kristalle, Ausb. 86.5 g (84%).
Schmp.: 185°C (MeOH).

Analog wurden hergestellt:

### Beispiel 2

### Bis(5-methoxy-N-phenylsulfonylindol-2-yl)-1-methanol

Schmp.: 113 - 114°C (MeOH)

### Beispiel 3

### (5-Methoxy-N-phenylsulfonylindol-2-yl)-(N-phenyl-sulfonylindol-2-yl)-1-methanol

Schmp.: 104 - 105°C (CH₂Cl₂ / Hexan)

### Beispiel 4

### (5-Methoxy-N-phenylsulfonylindol-2-yl)-(7-methoxy-N-phenylsulfonylindol-2-yl)-1-methanol

Schmp.: 119 - 121°C (CH₂Cl₂ / Hexan)

### Beispiel 5

### (7-Methoxy-N-phenylsulfonylindol-2-yl)-(N-phenylsulfonylindol-2-yl)-1-methanol

Schmp.: 99 - 101°C (CH₂Cl₂ / Hexan)

### Beispiel 6

### 5-Methoxy-2-phenylmethyloxy(1-phenylsulfonylindol-2-yl)methyl -1-phenylsulfonylindol

Schmp.: 62 - 64°C

### Beispiel 7

### Di-(5-Methyloxy-1-phenylsulfonylindol-2-yl)phenylmethyloxymethan

Schmp.: 100-101 °C

### Beispiel 8

### (3-Dimethylaminomethyl-1-phenylsulfonylindol-2-yl)(1-phenylsulfonylindol-2-yl)methan-1-ol

Schmp.: 116 - 117 °C

### Beispiel 9

### (7-Methoxy-N-phenylsulfonylindol-2-yl)-(N-phenyl-sulfonylindol-2-yl)-1-methanol

Schmp.: 149 - 151 °C

### Beispiel 10

### Dibenzothiophen-2-yl-1-methanol

Schmp.: 130 - 131 °C

### Beispiel 11

### 6-Methoxy-1-phenylsulfonyl-1H-2-indolyl(1-phenylsulfonyl-1H-2-indolyl)methanol

Schmp.: 180 °C

### Beispiel 12

### 7-Methoxy-1-phenylsulfonyl-1H-2-indolyl(1-phenylsulfonyl-1H-2-indolyl)methanol

Schmp.: 148 - 150 °C

### Beispiel 13

### Benzo[b]thiophen-2-yl(5-methoxy-1-phenylsulfonyl-1H-2-indolyl)-1-methanol

Schmp.: 71 - 73 °C

### Beispiel 14

### Benzo[b]thiophen-2-yl(7-methoxy-1-phenylsulfonyl-1H-2-indolyl)-1-methanol

Schmp.: 118-119 °C

### Beispiel 15

### Benzo[b]furan-2-yl(5-methoxy-1-phenylsulfonyl-1H-2-indolyl)-1-methanol

Schmp.: 71 - 73 °C

### Beispiel 16

### Bis(N-phenylsulfonylindol-2-yl)methan-1-on

Die Lösung von 20.00 g (36.9 mmol) Bis(*N*-phenylsulfonylindol-2-yl)-1-methanol in 200 ml absol. DMF wird auf 0°C gekühlt. Nach Zugabe von 90.4 g Pyridiniumdichromat (PDC) wird 20 h bei Raumtemp. gerührt. Zur Aufarbeitung werden 700 ml H₂O und 700 ml CH₂Cl₂ zugegeben. Die wäßr. Phase wird mit 2 x 200 ml CH₂Cl₂ extrahiert. Die vereinigten org. Extrakte werden mit 500 ml H₂O gewaschen. Nach Abziehen des Lösungsmittels i. Vak. und Zugabe von CH₂Cl₂ fällt das Produkt aus: farblose Kristalle, Ausb. 15.0 g (75%).
Schmp.: 244°C (MeOH / Ether)

Analog wurden hergestellt:

### Beispiel 17

### (5-Methoxy-N-phenylsulfonylindol-2-yl)-(N-phenylsulfonylindol-2-yl)methan-1-on

Schmp. 205°C (MeOH)

### Beispiel 18

### Bis(5-methoxy-N-phenylsulfonylindol-2-yl)-1-methanon

Schmp.: 190 - 191 °C

### Beispiel 19

### Bisindol-2-ylmethan-1-on

10.0 g (18.5 mmol) Bis(*N*-phenylsulfonylindol-2-yl)methan-1-on werden in 380 ml 99 proz. EtOH gelöst. Nach Zugabe von 210 ml 10 proz. NaOH wird die Lösung 20 h unter Rückfluß erhitzt. Zur Aufarbeitung wird das EtOH abgezogen, 500 ml ges. NaCl-Lösung und 500 ml CH₂Cl₂ werden zugefügt und die Phasen getrennt. Die wäßr. Phase wird mit 2 x 200 ml CH₂Cl₂ extrahiert, die vereinigten org. Extrakte werden über Na₂SO₄ getrocknet und i. Vak. eingeengt. Das Bisindol fällt als Rohprodukt aus und kann aus CH₂Cl₂ umkristallisiert werden, gelbe Kristalle, Ausb. 4.5 g (93%).
Schmp.: 272 - 273°C (CH₂Cl₂)

Analog wurden hergestellt:

### Beispiel 20

### (5-Methoxyindol-2-yl)-(indol-2-yl)methan-1-on

Schmp.: 233 - 235°C (MeOH)

### Beispiel 21

### Bis(5-methoxyindol-2-yl)-1-methanon

Schmp.: 202 - 204°C

### Beispiel 22

### Dibenzothiophen-2-yl-1-methanon

Schmp.: 161 °C

### Beispiel 23

### 5-Methoxy-1-phenylsulfonyl-3-indolyl(1-phenylsulfonyl-2-indolyl)-1-methanon

Schmp.: 114 - 116 °C

### Beispiel 24

### (1H-Indol-2-yl)-(1-H-indol-3-yl)-1-methanon

Schmp.: 260 - 261 °C (MeOH)

### Beispiel 25

### Benzo[b]thiophen-2-yl(7-methoxy-1-phenylsulfonyl-1H-2-indolyl)-1-methanon

Schmp.: 190 °C

### Beispiel 26

### Benzo[b]thiophen-2-yl(7-methoxy-1H-2-indolyl)-1-methanon

Schmp.: 155 °C

### Beispiel 27

### Benzo[b]thiophen-2-yl(5-methoxy-1-phenylsulfonyl-1H-2-indolyl)-1-methanon

Schmp.: 82 - 83 °C

### Beispiel 28

### Benzo[b]thiophen-2-yl(5-methoxy-1H-2-indolyl)-1-methanon

Schmp.: 200 °C

### Beispiel 29

### 7-Methoxy-1-phenylsulfonyl-1H-2-indolyl(1-phenylsulfonyl-1H-2-indolyl)methanon

Schmp.: 129 - 130 °C

### Beispiel 30

### 7-Methoxy-1H-2-indolyl(1H-2-indolyl)methanon

Schmp.: 151 °C

### Beispiel 31

### 6-Methoxy-1-phenylsulfonyl-1H-2-indolyl(1-phenylsulfonyl-1H-2-indolyl)methanon

Schmp.: 184 - 186 °C

### Beispiel 32

### 6-Methoxy-1H-2-indolyl(1H-2-indolyl)methanon

Schmp.: 184 - 186 °C

### Beispiel 33

### 1-Methyl-1H-2-indolyl(1-ethyl-5-methyloxy-1H-2-indolyl)-1-methanon

Schmp.: 148 - 149 °C

### Beispiel 34

### 1H-2-Indolyl(1-methyl-5-methyloxy-1H-2-indolyl)-1-methanon

Schmp.: 190 °C

### Beispiel 35

### 1-Methyl-1H-2-indolyl(5-methyloxy-1H-2-indolyl)-1-methanon

Schmp.: 176 - 177°C

### Beispiel 36

### 1-Ethyl-1H-2-indolyl(1-ethyl-5-methyloxy-1H-2-indolyl)-1-methanon

Schmp.: 99-100 °C

### Beispiel 37

### 1H-2-Indolyl(1-ethyl-5-methyloxy-1H-2-indolyl)-1-methanon

Schmp.: 142 - 143 °C

### Beispiel 38

### 1-Ethyl-1H-2-indolyl(5-methyloxy-1H-2-indolyl)-1-methanon

Schmp.: 101 - 102 °C

### Beispiel 39

### 1-Benzyl-1H-2-indolyl(1-benzyl-5-methoxy-1H-2-indolyl)-1-methanon

Schmp.: 132 °C

### Beispiel 40

### 1H-2-Indolyl(1-benzyl-5-methoxy-1H-2-indolyl)-1-methanon

Schmp.: 180 - 182 °C

### Beispiel 41

### 1-Benzyl-1H-2-indolyl(5-methoxy-1H-2-indolyl)-1-methanon

Schmp.: 167 - 168 °C

### Beispiel 42

### 5-Benzyloxy-1H-2-indolyl(1H-2-indolyl)methanon

Schmp.: 199 - 201 °C.

### Beispiel 43

### 5-Hydroxy-1H-2-indolyl(1H-2-indolyl)methanon

Schmp.: > 220 °C.

### Beispiel 44

### 5-Ethoxy-1H-2-indolyl(1H-2-indolyl)methanon

Schmp.: 168 - 169 °C.

### Beispiel 45

### 1H-2-Indolyl[5-(2-morpholin-1-ylethyloxy)-1H-2-indolyl]methanon

Schmp.: 98 - 101 °C.

### Beispiel 46

### 1H-2-lndolyl[5-(3-dimethylaminopropyloxy)-1H-2-indolyl]methanon

Schmp.: 163 - 166 °C.

### Beispiel 47

### 5-(4-Iodobutyloxy)-1H-2-indolyl(1H-2-indolyl)methanon

Schmp.: 110 - 113 °C.

### Beispiel 48

### 1H-2-Indolyl[5-(2-dimethylaminoethyloxy)-1H-2-indolyl]methanon

Schmp.: 143 - 145 °C.

### Beispiel 49

### 5-Cyclohexylmethyloxy-1H-2-indolyl(1H-2-indolyl)methanon

Schmp.: 185 °C (Zers.).

### Beispiel 50

### 5-(5-Iodopentyloxy)-1H-2-indolyl(1H-2-indolyl)methanon

Schmp.: 127-130 °C.

### Beispiel 51

### 1-H-2-lndolyl[5-(1-phenylethyloxy)-1H-2-indolyl]methanon

Schmp.: 151 - 153 °C.

### Beispiel 52

### 1H-2-Indolyl[5-(2-piperidin-1-ylethyloxy)-1H-2-indolyl]methanon

Schmp.: 104 - 106 °C.

### Beispiel 53

### [2-(1H-2-Indolylcarbonyl)-1H-5-indolyl)] ethanoat

Schmp.: 223 - 224 °C.

### Beispiel 54

### [2-(1H-2-Indolylcarbonyl)-1H-5-indolyl)] 4-methoxybenzoat

Schmp.: > 230 °C.

### Beispiel 55

### [2-(1H-2-Indolylcarbonyl)-1H-5-indolyl)] butanoat

Schmp.: 201 - 204 °C.

### Beispiel 56

### [2-(1H-2-Indolylcarbonyl)-1H-5-indolyl)] 2-(N,N)-dimethylaminoethanoat

Schmp.: 215 - 217 °C.

### Beispiel 57

### [2-(1H-2-Indolylcarbonyl)-1H-5-indolyl)] propanoat

Schmp.: > 230 °C.

### Beispiel 58

### [2-(1H-2-Indolylcarbonyl)-1H-5-indolyl)] 2-thiophenylethanoat

Schmp.: 224 - 226 °C.

### Beispiel 59

### [2-(1H-2-Indolylcarbonyl)-1H-5-indolyl)] O-acetylsalycylat

Schmp.: 133 - 135 °C.

### Beispiel 60

### [2-(1H-2-indolylcarbonyl)-1H-5-indolyl)]-4-phenylbenzoat

Schmp.: >220 °C.

### Beispiel 61

### [2-(1H-2-Indolylcarbonyl)-1H-5-indolyl)] 2-phenylpropanoat

Schmp.: 211 - 313 °C.

### Beispiel 62

### [2-(1H-2-Indolylcarbonyl)-1H-5-indolyl)] α-acetylphenylethanoat

Schmp.: 194 - 196 °C.

### Beispiel 63

### [2-(1H-2-Indolylcarbonyl)-1H-5-indolyl)] benzoat

Schmp.: > 230 °C.

### Beispiel 64

### [2-(1H-2-Indolylcarbonyl)-1H-5-indolyl)]3-methoxyphenylethanoat

Schmp.: 212 - 215 °C.

### Beispiel 65

### [2-(1H-2-lndolylcarbonyl)-1H-5-indolyl)] 2-chlorobenzoat

Schmp.: > 230 °C.

### Beispiel 66

### [2-(1H-2-Indolylcarbonyl)-1H-5-indolyl)] 4-nitrobenzoat

Schmp.: > 230 °C.

### Beispiel 67

### [2-(1H-2-Indolylcarbonyl)-1H-5-indolyl)]3,4,5-trimethoxybenzoat

Schmp.: 216 - 219 °C.

### Beispiel 68

### [2-(1H-2-lndolylcarbonyl)-1H-5-indolyl)] cinnamat

Schmp.: 226 - 228 °C.

### Beispiel 69

### [2-(1H-2-Indolylcarbonyl)-1H-5-indolyl)] 2-furanylcarboxylat

Schmp.: > 230 °C.

### Beispiel 70

### Di(1-phenylsulfonyl-1H-2-indolyl)methan

Zu einer Lösung von 26.67 g (49.2 mmol) Bis(*N*-phenylsulfonylindol-2-yl)-1-methanol und 15.00 g (57.8 mmol) Triphenylsilan in 400 ml absol. CH₂Cl₂ wird nach 30 min 22.4 ml Trifluoressigsäure (TFA) getropft. Nach 1 h Rühren bei Raumtemp. wird H₂O zugegeben und die Mischung unter Eiskühlung vorsichtig mit festem Na₂CO₃ neutralisiert. Nach Trennung der Phasen, Trocknen der org. Phase über Na₂SO₄ und Abdestillieren des Lösungsmittels wird das Rohprodukt sc (SiO₂; CH₂Cl₂ / Hexan 6:4) gereinigt, farblose Kristalle, Ausb. 22.5 g (87%).
Schmp.: 144 - 145°C (Ether)

Analog wurden hergestellt:

### Beispiel 71

### Bis(5-methoxy-N-phenylsulfonylindol-2-yl)methan

Schmp.: 159 - 160°C (CH₂Cl₂ / Hexan).

### Beispiel 72

### (5-Methoxy-N-phenylsulfonyl-indol-2-yl)-(N-phenyl-sulfonyl-indol-2-yl)methan

Schmp.: 98 - 100°C (CH₂Cl₂ / Hexan).

### Beispiel 73

### (5-Methoxy-N-phenylsulfonylindol-2-yl)-(7-methoxy-N-phenylsulfonylindol-2-yl)methan

Schmp.: 168 -170°C (CH₂Cl₂ / Hexan)

### Beispiel 74

### Di(1H-2-indolyl)methan

15.0 g (28.5 mmol) 57 werden mit 20 g K₂CO₃ in 800 ml MeOH und 200 ml H₂O 14 Tage gekocht. Zur Aufarbeitung werden 500 ml ges. NaCI-Lösung zugegeben und die Phasen getrennt. Nach dem Trocknen der org. Phase wird das Lösungsmittel i. Vak. abgezogen. Das Rohprodukt wird sc gereinigt, farblose Kristalle, Ausb. 5.4 g (76%).
Schmp.: 189 - 191°C

Analog wurden hergestellt:

### Beispiel 75

### (5-Methoxyindol-2-yl)-(indol-2-yl)methan

Schmp.: 112°C (MeOH).

### Beispiel 76

### (1H-Indol-2-yl)-(1-H-indol-3-yl)-1-methan

Schmp.: 161 - 163 °C (aq. EtOH)

### Beispiel 77

### 1,3-Di(1H-2-indolyl)propan

Man löst 38.0 g (0.21 mol) Trimethylsilyl-o-toluidid in 950 ml abs. Hexan und tropft bei Raumtemp. 291.0 ml (0.47 mol) *n*-BuLi (1.6 M in Hexan) zu und erhitzt die Mischung 4 h lang zum Rückfluß. Anschließend kühlt man auf - 78 °C ab und tropft bei dieser Temp. 20.5 ml (0.11 mol) Glutarsäurediethylester in 380 ml abs. THF zu. Man rührt 1 h bei- 78 °C, läßt dann langsam über Nacht auf Raumtemp. kommen und erhitzt anschließend noch 2 h zum Sieden. Nach dem Abkühlen gießt man auf 1 I Eiswasser und extrahiert mit 5 * 500 ml Essigester, trocknet die vereinigten org. Phasen über Na₂SO₄ und zieht das Lösungsmitel *i*. *Vak*. ab. Weiße Kristalle, Ausb.: 6.55 g (23.9 mmol, 22 %).
Schmp.: 143 - 145 °C (Ethanol)

Analog wurde hergestellt:

### Beispiel 78

### 1,3-Di(1H-2-indolyl)ethan

Schmp.: 264 - 267 °C

### Beispiel 79

### 1,2-Di-(1-phenylsulfonyl-1H-2-indolyl)-1-ethen

(17.9 mmol) TiCl₄ mit einer Spritze und gibt anschließend 2.0 g (30.5 mmol) Zn-Pulver dazu. Der Ansatz wird 30 min unter Rückfluß erhitzt. Danach tropft man wieder bei 0 °C 3 g (10.5 mmol) **22,** gelöst in 50 ml THF zu. Die Lösung wird über Nacht unter Rückfluß erhitzt. Zu der abgekühlten Lösung gießt man 300 ml 20 proz. K₂CO₃ - Lsg. und läßt über Nacht die Raumtemp. weiterrühren. Der schlammige Rückstand wird dann abfiltriert und mit THF nachgewaschen, vom Filtrat wird die org. Phase abgetrennt, die wäßrige mit CH₂Cl₂ extrahiert.
Die vereinigten org. Phasen werden mit Wasser gewaschen, über Na₂SO₄ getrocknet und i. Vak. vom LM befreit. Die Reinigung erfolgt sc (SiO₂; CH₂Cl₂/Hexan 2:1). Ausbeute: 1.1 g (2.0 mmol, 39 %) gelbe Kristalle.
Schmp.: 272 °C

### Beispiel 80

### Bis(5-methoxy-N-phenylsulfonylindol-2-yl)phenoxymethan

Zu einer Lösung von 2 g (3.7 mmol) Bis(*N*-phenylsulfonylindol-2-yl)-1-methanol in 20 ml THF gibt man bei 0 °C 188 mg NaH (60 % in Paraffin). Anschließend fügt man 13. 5 mg Tetrabutylammoniumiodid und 0.45 ml Benzylbromid zu und rührt bei 20 °C. Anschließend gibt man vorsichtig Wasser und Ether zu, trennt den Ether ab und wäscht die wässrige Phase 2 mal mit Ether. Die org. Phase wird über Na₂SO₄ getrocknet und anschließend das Lösungsmittel abgezogen. Ausbeute: 0.86 mg (81 %)
Schmp.: 192 °C (Zers.)

### Beispiel 81

1,2,3,8,9,10-Hexahydroindolo[3',2':5,6]pyrrolo[3',4':3,4]-cyclohepta[*b*]indol-1,3-dion Zu 236 mg (9.75 mmol) Mg-Späne in 6 ml absol. THF gibt man die Hälfte von 0.73 ml (9.75 mmol) wasserfreiem Ethylbromid. Nach Anspringen der Reaktion tropft man den Rest des Ethylbromids so zu, daß die Lösung weiter siedet. Anschließend wird bis zur Auflösung der Mg-Späne gekocht (ca. 30 min). Nach Abkühlen auf Raumtemp. wird 1.00 g (4.06 mmol) Methylen-2,2'-bisindol in 25 ml absol. Toluol und 1 ml absol. THF zugetropft und 45 min bei 45 °C gerührt. Nach erneutem Abkühlen auf Raumtemp. werden 1.04 g (4.06 mmol) Dibrommaleinimid in 50 ml absol. Toluol und 2 ml absol. THF über 1 h zugetropft, dann wird über Nacht unter Rückfluß erhitzt. Zur Aufarbeitung werden 100 g Eis und 50 ml 20 pro.
Zitronensäure zugegeben, dann wird mit 2 x 50 ml Essigester ausgeschüttelt. Die org. Extrakte werden mit H₂O gewaschen, über Na₂SO₄ getrocknet und eingeengt. Das Rohprodukt wird sc (SiO₂, 1. CH₂Cl₂ / Essigester 8 : 2; 2. CH₂Cl₂ / Essigester 7 : 1) gereinigt: rote Kristalle, Ausb. 290 mg (22 %), Schmp.: > 350 °C (Essigester).

Analog wurden hergestellt:

### Beispiel 82

### 1,2,3,8,9,10-Hexahydro-5-methoxyindolo[3',2':5,6]pyrrolo(3',4':3,4]-cyclohepta[b]indol-1,3-dion

Schmp.: > 350 °C (EtOH)

### Beispiel 83

### 1,2,3,8,9,10,11,12-Octahydroindolo[3',2':5,6]pyrrolo[3',4':3,4]-cyclonona[b]indol-1,3-dion

Schmp.: 137 °C (CH₂Cl₂) (Zers.)

### Beispiel 84

### 1,2,3,8,9,10,11-Heptahydro-2-methylindolo[3',2':5,6]pyrrolo[3',4':3,4]-cycloocta[b]indol-1,3-dion

Schmp.: > 350 °C

### Beispiel 85

### 2-Benzyloxymethyl-1,2,3,8,9,10-Hexahydroindolo[3',2':5,6]pyrrolo[3',4':3,4]-cyclohepta[b]indol-1,3-dion

Schmp.: > 350 °C (EtOH)

### Beispiel 86

### 1,2,3,8,9,10-Hexahydro-2-methylindolo[3',2':5,6]pyrrolo[3',4':3,4]-cyclohepta[b]indol-1,3-dion

Schmp.: > 350 °C (CH₂Cl₂)

### Beispiel 87

### 3,8,9,10-Tetrahydro-8-[2-(N,N-dimethylamino)ethyl]-1H-indolo[3',2':5,6]furo-[3',4':3,4]cyclohepta[b]indol-1,3-dion

Schmp.: >350°C (MeOH)

### Beispiel 88

### 2-Benzyloxymethyl-1,2,3,8,9,10-hexahydro-8-[2-(N,N-dimethylamino)ethyl]indolo[3',2':5,6]pyrrolo[3',4':3,4]cyclohepta[b]indol-1,3-dion

Schmp.: 164 - 165°C (MeOH)

### Beispiel 89

### 1,2,3,8,9,10-Hexahydro-2-methyl-8-[2-(N,N-dimethylamino)ethyl]indolo[3',2':5,6]-pyrrolo[3',4':3,4]cyclohepta[b]indol-1,3-dion

Schmp.: 185°C (MeOH)

### Beispiel 90

### 1,2,3,8,9,10-Hexahydro-8-[2-(N,N-dimethylamino)ethyl]indolo[3',2':5,6]pyrrolo-[3',4':3,4]cyclohepta[b]indol-1,3-dion

Schmp.: 213 - 214°C (EtOH)

### Beispiel 91

### 3-Bromo-4-(2-(2-(1H-2-indolyl)ethyl)-1H-3-indolyl)-1-methyl-2,5-dihydro-1H-pyrrol-2,5-dion

Schmp.: 169 °C

### Beispiel 92

### 3-Bromo-4-(2-(4-(1H-2-indolyl)butyl)-1H-3-indolyl)-1-methyl-2,5-dihydro-1H-pyrrol-2,5-dion

Schmp.: 165 °C (Zers.)

### Beispiel 93

### 3-Bromo-4-(2-(5-(1H-2-indolyl)pentyl)-1H-3-indolyl)-1-methyl-2,5-dihydro-1H-pyrrol-2.5-dion

Schmp.: 125 °C (Zers.)

### Beispiel 94

### Bis(indol-3-yl)methanon

Analog Beispiel 31 mit Triphosgen anstelle von Dibrommaleinimid.
Smp.: 297 - 299 °C

### Beispiel 95

Diastereomerengmisch aus 8-(3,4,6-Tri-*O*-benzyl-β-D-glucopyranosyl)-2-benzyloxymethyl-1,2,3,8,9,10-hexahydroindolo[3',2':5,6]pyrrolo[3',4':3,4]cyclohepta[*b*]indol-1,3-dion und
8-(3,4,6-Tri-*O*-benzyl-α-D-mannopyranosyl)-2-benzyloxymethyl-1,2,3,8,9,10-hexahydroindolo[3',2':5,6]pyrrolo[3',4':3,4]cyclohepta[*b*]indol-1,3-dion Diastereomerengemisch der disubstituierten *O*-Glycoside

468.7 mg (1.02 mmol) 2-Benzyloxymethyl-1,2,3,8,9,10-hexahydroindolo[3',2':5,6]pyrrolo[3',4':3,4]cyclohepta[*b*]indol-1,3-dion werden zu einer Suspension von 91.8 mg (3.06 mmol) NaH (80 proz. in Paraffinöl) in 16 ml absol. THF gegeben. Nach 30 min wird die Lösung von 1,2-Anhydro-3,4,6-tri-*O*-benzyl-D-glucopyranose in 16 ml absol. THF zugetropft. Der Ansatz wird 5 h bei 50°C und 1 h bei 60°C gerührt. Die Reaktionslösung wird zur Aufarbeitung auf 10 ml ges. NaHCO₃-Lösung gegossen und mit 3 x 10 ml Essigester extrahiert. Die vereinigten org. Extrakte werden mit 15 ml ges. NaCI-Lösung gewaschen, über Na₂SO₄ getrocknet und i. Vak. eingeengt. Das Produkt wird sc (1. Säule: SiO₂; Toluol / Isopropylamin 8 : 2; 2. Säule: SiO₂; CH₂Cl₂ / MeOH 12 : 1) von Nebenprodukten und nicht umgesetztem Edukt getrennt. Die Trennung des Diastereomerengemisches erfolgt durch HPLC.

### Beispiel 96

Diastereomerengemisch aus 8-(β-D-Glucopyranosyl)-1,2,3,8,9,10-hexahydroindolo [3',2':5,6]pyrrolo[3',4':3,4]cyclohepta[*b*]indol-1,3-dion und 8-(α-D-Mannopyranosyl)-1,2,3,8,9,10-hexahydroindolo[3',2':5,6]pyrrolo[3',4':3,4]cyclohepta[*b*]-indol-1,3-dion

150 mg (0.17 mmol) 8-(3,4,6-Tri-*O*-benzyl-D-glucopyranosyl)-2-benzyloxymethyl-1,2,3,8,9,10-hexahydroindolo[3',2':5,6]pyrrolo[3',4':3,4]cyclohepta[*b*]indol-1,3-dion, als Diastereomerengemisch, werden in 50 ml absol. EtOH gelöst und nach der Zugabe von 200 mg Pd / C (5%) 5 h unter 7 bar H2-Druck gerührt. Danach wird über Celite abgesaugt, mit 50 ml CH₂Cl₂ nachgespült und die Lösung i. Vak. eingeengt. Ohne Reinigung wird das Rohprodukt in 15 ml absol. THF gelöst und die Lösung auf 0°C abgekühlt. Dann wird 10 min lang NH₃ eingeleitet und 1 h bei Raumtemp. gerührt. Nach Abziehen des THF i. Väk. wird das verbleibende Öl sc (SiO₂; CH₂Cl₂ / MeOH 8 : 2) gereinigt: rotes Öl, Ausb. 10 mg (12%).

### Beispiel 97

### 1,2,3,3a,8,9,10,14c-Octahydroindolo[3',2':5,6]pyrrolo[3',4':3,4]cyclohepta[b]indol-1,3-dion

1.20 g (18.4 mmol) Zn - Granulat werden mit 2 x 3 ml 2 N HCI gewaschen, anschließend sofort zu 90 mg (0.33 mmol) HgCl₂ in 1.5 ml H₂O und 1.5 ml konz. HCl gegeben und 10 min bei Raumtemp. geschüttelt. Die wäßr. Phase wird dekantiert und das Zinkamalgam noch mit 2 x 3 ml verd. HCI gewaschen, bevor es zu einer Lösung von 60.0 mg (0.18 mmol) 1,2,3,8,9,10-Hexahydroindolo[3',2':5,6]pyrrolo[3',4':3,4]cyclohepta[*b*]indol-1,3-dion in 1.5 ml 5N HCl, 1.5 ml EtOH und 1.5 ml Toluol gegeben und unter Rückfluß erhitzt wird. Nach 1 h wird, sobald die Reaktionslösung auf Raumtemp. abgekühlt ist, H₂O zugegeben und mit 2 x 10 ml CH₂Cl₂ extrahiert. Die org. Extrakte werden über Na₂SO₄ getrocknet, i. Vak. eingeengt und sc (SiO₂; CH₂Cl₂ / Essigester / MeOH 8 : 2 : 0.5) gereinigt: farbloses Wachs, Ausb 14 mg (23%).

### Beispiel 98

### 2,5-Dihydro-3,4-bis(N-trimethylsilylethoxymethylindol-2-yl)-1H-pyrrolo-2,5-dion

Zu einer Lösung von 22.65 mg (0.02 mmol) Tetrakistriphenylphosphinpalladium und 450.0 mg (1.77 mmol) 3,4-Dibromo-2,5-dihydro-1*H*-pyrrolo-2,5-dion in 10 ml absol. DMF tropft man 1.05 g (1.96 mmol) 2-Tributylstannyl-*N*-trimethylsilylethoxymethylindol in 5 ml absol. DMF und erhitzt anschließend 1 h auf 110°C. Nachdem dem Abkühlen wird auf 50 ml H₂O gegossen und mit 2 x 50 ml Ether extrahiert. Die Etherphasen werden mit 100 ml H₂O gewaschen, über Na₂SO₄ getrocknet und eingeengt. Durch SC (1. Säule: SiO₂; CH₂Cl₂ / MeOH / Hexan 20 : 1 : 2, 2. Säule: SiO₂; CH₂Cl₂ / Essigester 20 : 1) können die Produkte getrennt werden.
gelbes Wachs, Ausb. 200 mg (19%).

Analog wurden hergestellt:

### Beispiel 99

### 2,5-Dihydro-3,4-bisindol-2-yl-1H-pyrrolo-2,5-dion

Schmp.: 197°C (Zers.) (CH₂Cl₂ / Hexan)

### Beispiel 100

### 2,5-Dihydro-3,4-bis(N-phenylsulfonylindol-2-yl)-1H-pyrrolo-2,5-dion

Schmp.: 196 - 197°C (Zers.) (Aceton)

### Beispiel 101

### 2,5-Dihydro-1-methyl-3,4-bis(N-phenylsulfonylindol-2-yl)-1H-pyrrolo-2,5-dion

Schmp.: 147°C (Ether)

### Beispiel 102

### 2,5-Dihydro-3,4-bisindol-2-yl-1-methyl-1H-pyrrolo-2,5-dion

Schmp.: 247°C (CH₂Cl₂ / Hexan) (Zers.)

### Beispiel 103

### 2,5-Dihydro-3-indol-2-yl-1-[2-(N,N-dimethylamino)ethyl]-4-(N-phenylsulfonylindol-2-yl)-1H-pyrrolo-2,5-dion

### 2,5-Dihydro-1-[2-(N,N-dimethylamino)ethyl]-3,4-bis(N-phenylsutfonylindol-2-yl)-1Hpyrrolo-2,5-dion

4.12 mmol 2,5-Dihydro-3,4-bis(*N*-phenylsulfonylindol-2-yl)-1*H*-pyrrolo-2,5-dion werden in 30 ml absol. DMF gelöst, und unter Rühren werden vorsichtig 200 mg (5.00 mmol) KH zugegeben. Nach 1 h Rühren bei Raumtemp. wird das Halogenid zugesetzt und 24 h bei Raumtemp. gerührt. Zur Aufarbeitung wird der Ansatz auf Eiswasser gegossen. DMF und H₂O werden im Vak. abdestilliert, der Rückstand wird in CH₂Cl₂ gelöst und mit H₂O gewaschen. Nach dem Trocknen über Na₂SO₄ wird das LM i. Vak. abgezogen und der Rückstand durch SC (SiO₂; Essigester) gereinigt. Ausb. 448 mg. 121 und 122 ließen sich durch SC nicht trennen.

Analog wurden erhalten:

### Beispiel 104

### 2,5-Dihydro-3,4-bis(indol-2-yl)-1[2-(N,N-dimethylamino)ethyl]-1H-pyrrolo-2,5-dion oranges Wachs

### Beispiel 105

### 1-(2-Bromethyl)-2,5-dihydro-3,4-bis(N-phenylsulfonylindol-2-yl)-1H-pyrrolo-2,5-dion gelb-braunes Wachs

### Beispiel 106

### 1-(2-Bromethyl)-2,5-dihydro-3-indol-2-yl-4-(N-phenylsulfonylindol-2-yl)-1H-pyrrolo-2,5-dion

Schmp.: 160°C (Zers.)

### Beispiel 107

### 1-(2-Bromethyl)-2,5-dihydro-3,4-bis(indol-2-yl)-1H-pyrrolo-2,5-dion

Schmp.: 104 - 109 °C

### Beispiel 108

### 1-(2-Azidoethyl)-2,5-dihydro-3,4-bis(N-phenylsulfonylindol-2-yl)-1H-pyrrolo-2,5-dion

Schmp.: 165 °C (Zers.)

### Beispiel 109

### 1-(2-Azidoethyl)-2,5-dihydro-3-indol-2-yl-4-(N-phenylsulfonylindol-2-yl)-1H-pyrrolo-2,5-dion

Schmp.: 190 °C (Zers.)

### Beispiel 110

### 1-(2-Aminoethyl)-2,5-dihydro-3-indol-2-yl-4-(N-phenylsulfonylindol-2-yl)-1H-pyrrolo-2,5-dion

Schmp.: 180 °C (Zers.)

### Beispiel 111

### 3-Bromo-4-(2-(3-(3-(4-bromo-1-methyl-2,5-dioxo-2,5-dihydro-1H-3-pyrrolyl)-1H-2-indolyl)propyl)-1H-3-indolyl)-1-methyl-2,5-dihydro-1H-pyrrol-2,5-dion

200 mg (0.7 mmol) 1,3-Di(1*H*-2-indolyl)propan werden in 4 ml absol. THF gelöst und auf 0 °C abgekühlt. Dann tropft man 1,09 ml (1.7 mmol) *n*-BuLi (1.6 M in Hexan) während 30 min zu und rührt die Mischung 2 h bei Raumtemp.. Anschließend tropft man 0.46 g (1.71 mmol) *N*-Methyldibrommaleinimid in 4 ml absol. THF langsam zu. Der Ansatz wird über Nacht bei Raumtemp. gerührt und dann auf 10 ml 2 N HCl gegossen. Die Mischung wird dann mit Ether (2 x 10 ml) und Essigester (3 x 10 ml) extrahiert, die org. Phase über Na₂SO₄ getrocknet und das Lösungsmittel *i. Vak.* abgezogen. Der Rückstand wird sc (SiO₂, CH₂Cl₂) gereinigt. Rotes Pulver, Ausb.: 0.20 g (44 %).
Schmp.: 160 °C (Zers.)

Analog wurden hergestellt:

### Beispiel 112

### 3-Bromo-4-(2-(5-(3-(4-bromo-1-methyl-2,5-dioxo-2,5-dihydro-1H-3-pyrrolyl)-1H-2-indolyl)pentyl)-1H-3-indolyl)-1-methyl-2,5-dihydro-1H-pyrrol-2,5-dion

Schmp.: 137 °C (Zers.)

### Beispiel 113

### 3-Bromo-4-(2-(3-(3-(4-bromo-2,5-dioxo-2,5-dihydro-1H-3-pyrrolyl)-1H-2-indolyl)propyl)-1H-3-indolyl)-2,5-dihydro-1H-pyrrol-2,5-dion

Schmp.: > 350 °C

### Beispiel 114

### 3-Bromo-4-(2-(5-(3-(4-bromo-2,5-dioxo-2,5-dihydro-1H-3-pyrrolyl)-1H-2-indolyl)pentyl)-1H-3-indolyl)-2,5-dihydro-1H-pyrrol-2,5-dion

Schmp.: > 350 °C

### Beispiel 115

### 3-Bromo-4-(2-(8-(3-(4-bromo-2,5-dioxo-2,5-dihydro-1H-3-pyrrolyl)-1H-2-indolyl)octyl)-1H-3-indolyl)-2,5-dihydro-1H-pyrrol-2,5-dion

Schmp.: 180 °C (Zers.)

### Beispiel 116

### 3-Bromo-4-(2-(2-(3-(4-bromo-1-methyl-2,5-dioxo-2,5-dihydro-1H-3-pyrrolyl)-1H-2-indolyl)ethyl)-1H-3-indolyl)-1-methyl-2,5-dihydro-1H-pyrrol-2,5-dion

Schmp.: 179 °C

### Beispiel 117

### 3-Bromo-4-(2-(4-(3-(4-bromo-1-methyl-2,5-dioxo-2,5-dihydro-1H-3-pyrrolyl)-1H-2-indolyl)butyl)-1H-3-indolyl)-1-methyl-2,5-dihydro-1H-pyrrol-2,5-dion

Schmp.: 190 °C (Zers.)

### Beispiel 118

### 3-Bromo-4-(2-(8-(3-(4-bromo-1-methyl-2,5-dioxo-2,5-dihydro-1H-3-pyrrolyl)-1H-2-indolyl)octyl)-1H-3-indolyl)-1-methyl-2,5-dihydro-1H-pyrrol-2,5-dion

Schmp.: 185 °C (Zers.)

### Beispiel 119

### 3-Bromo-4-(2-(10-(3-(4-bromo-1-methyl-2,5-dioxo-2,5-dihydro-1H-3-pyrrolyl)-1H-2-indolyl)decyl)-1H-3-indolyl)-1-methyl-2,5-dihydro-1H-pyrrol-2,5-dion

Schmp.: 164 °C (Zers.)

### Beispiel 120

### 3-Bromo-4-(2-(10-(3-(4-bromo-2,5-dioxo-2,5-dihydro-1H-3-pyrrolyl)-1H-2-indolyl)decyl)-1H-3-indolyl)-2,5-dihydro-1H-pyrrol-2,5-dion

Schmp.: 164 °C (Zers.)

### Beispiel 121

### 3-Bromo-4-(2-(12-(3-(4-bromo-1-methyl-2,5-dioxo-2,5-dihydro-1H-3-pyrrolyl)-1H-2-indolyl)dodecyl)-1H-3-indolyl)-1-methyl-2,5-dihydro-1H-pyrrol-2,5-dion

Schmp.: 126 - 129 °C

Durch Umsetzung der Verbindung von Beispiel 114 mit Dimethylamin wurde erhalten:

### Beispiel 122

### 3-N,N-dimethyl-amino-4-(2-(5-(3-(4-N,N-dimethyl-amino-2,5-dioxo-2,5-dihydro-1H-3-pyrrolyl)-1H-2-indolyl)pentyl)-1H-3-indolyl)-2,5-dihydro-1H-pyrrol-2,5-dion

### Beispiel 123

### 1-Methyl-3-(1-pyrrolidinyl)-4-(2-(5-(3-(1-methyl-4-(1-pyrrolidinyl)-2,5-dioxo-2,5-dihydro-1H-3-pyrrolyl)-1H-2-indolyl)pentyl)-1H-3-indolyl)-2,5-dihydro-1H-pyrrol-2,5-dion

Man löst 1.0 g (1.5 mmol) 3-Bromo-4-(2-(5-(3-(4-bromo-1-methyl-2,5-dioxo-2,5-dihydro-1*H*-3-pyrrolyl)-1*H*-2-indolyl)pentyl)-1*H*-3-indolyl)-1-methyl-2,5-dihydro-1*H*-pyrrol-2,5-dion in 5 ml (60.6 mmol) Pyrrolidin und läßt über Nacht bei Raumtemp. rühren. Dann wird überschüssiges Pyrrolidin abdestilliert. Der Rückstand wird vollständig von Lösungsmittelresten im Ölpumpenvakuum befreit und anschließend sc (SiO₂, CH₂Cl₂ / Essigester 95 : 5) gereinigt. Ausb.: 480 mg (49 %).
Schmp.: 298 °C

Analog wurden hergestellt:

### Beispiel 124

### 1-Methyl-3-(1-piperidinyl)-4-(2-(5-(3-(1-methyl-4-(1-piperidinyl)-2,5-dioxo-2,5-dihydro-1H-3-pyrrolyl)-1H-2-indolyl)pentyl)-1H-3-indolyl)-2,5-dihydro-1H-pyrrol-2,5-dion

Schmp.: 262 °C

### Beispiel 125

### 1-Methyl-3-(1-morpholinyl)-4-(2-(5-(3-(1-methyl-4-(1-morpholinyl)-2,5-dioxo-2,5-dihydro-1H-3-pyrrolyl)-1H-2-indolyl)pentyl)-1H-3-indolyl)-2,5-dihydro-1H-pyrrol-2,5-dion

Schmp.: 168 - 170 °C

### Beispiel 126

### 1-Methyl-3-(1-tetrahydrolsochinolinyl)4-(2-(5-(3-(1-methyl-4-(1-tetrahydroisochinolinyl)-2,5-dioxo-2,5-dihydro-1H-3-pyrrolyl)-1H-2-indolyl)pentyl)-1H-3-indolyl)-2,5-dihydro-1H-pyrrol-2,5-dion

Schmp.: 141 - 142 °C

### Beispiel 127

### 1-Methyl-3-(1-(4-(3-trifluormethylphenyl)piperazinyl))-4-(2-(5-(3-(1-methyl-4-(1-(4-(3-trifluormethylphenyl)piperazinyl))-2,5-dioxo-2,5-dihydro-1H-3-pyrrolyl)-1H-2-indolyl)pentyl)-1H-3-indolyl)-2,5-dihydro-1H-pyrrol-2,5-dion

Schmp.: 140 -141 °C

### Beispiel 128

### 1-Methyl-3-(1-(4-isopropylaminocarbonylmethylpiperazinyl))-4-(2-(5-(3-(1-methyl-4-(1-(4-isopropylaminocarbonylmethylpiperazinyl))-2,5-diox-2,5-dihydro-1H-3-pyrrolyl)-1H-2-indolyl)pentyl)-1H-3-indolyl)-2,5-dihydro-1H-pyrrol-2,5-dion

Schmp.: 126 - 128 °C

### Beispiel 129

### 1-Methyl-3-(1-(4-isopropylaminocarbonylmethylpiperazinyl))-4-(2-(5-(3-(4-bromo-1-methyl-2,5-dioxo-2,5-dihydro-1H-3-pyrrolyl)-1H-2-indolyl)pentyl)-1H-3-indolyl)-2,5-dihydro-1H-pyrrol-2,5-dion

Schmp.: 156 °C

### Beispiel 130

### 1-Methyl-3-(1-(4-pyrrolidinylcarbonylmethylpiperazinyl))-4-(2-(5-(3-(1-methyl-4-(1-(4-pyrrolidinylcarbonylmethylpiperazinyl))-2,5-dioxo-2,5-dihydro-1H-3-pyrrolyl)-1H-2-indolyl)pentyl)-1H-3-indolyl)-2,5-dihydro-1H-pyrrol-2,5-dion

Schmp.: 158 °C (Zers.)

### Beispiel 131

### 1-Methyl-3-(1-(4-pyrrolidinylcarbonylmethylpiperazinyl))-4-(2-(5-(3-(4-bromo-1-methyl-2,5-dioxo-2,5-dihydro-1H-3-pyrrolyl)-1H-2-indolyl)pentyl)-1H-3-indolyl)-2,5-dihydro-1H-pyrrol-2,5-dion

Schmp.: 158 - 159 °C

### Beispiel 132

### 1-Methyl-3-(1-(4-piperidinopiperidinyl))-4-(2-(5-(3-(1-methyl-4-(1-(4-piperidinopiperidinyl))-2,5-dioxo-2,5-dihydro-1H-3-pyrrolyl)-1H-2-indolyl)pentyl)-1H-3-indolyl)-2,5-dihydro-1H-pyrrol-2,5-dion

Schmp.: 230 - 232 °C (Zers.)

### Beispiel 133

### 1-Methyl-3-(1-(4-piperidinopiperidinyl))-4-(2-(5-(3-(4-bromo-1-methyl-2,5-dioxo-2,5-dihydro-1H-3-pyrrolyl)-1H-2-indolyl)pentyl)-1H-3-indolyl)-2,5-dihydro-1H-pyrrol-2,5-dion

Schmp.: 162 - 164 °C

### Beispiel 134

### 1-Methyl-3-(1-(4-ethoxycarbonylpipearzin-1-yl))-4-(2-(5-(3-(1-methyl-(4-ethoxycarbonylpiperazin-1-yl)-2,5-dioxo-2,5-dihydro-1H-3-pyrrolyl)-1H-2-indolyl)pentyl)-1H-3-indolyl)-2,5-dihydro-1H-pyrrol-2,5-dion

Schmp.: 149 - 150 °C

### Beispiel 135

### 1-Methyl-3-(1-(4-(N-(4-hydroxyphenyl)-ethylamin))-4-(2-(5-(3-(1-methyl-(4-bromo-2,5-dioxo-2,5-dihydro-1H-3-pyrrolyl)-1H-2-indolyl)pentyl)-1H-3-indolyl)-2,5-dihydro-1H-pyrrol-2,5-dion

Schmp.: 120 - 122 °C (Zers.)

### Beispiel 136

### 1-Methyl-3-(1-(4-(N-1,2-diaminoethyl))-4-(2-(4-(3-(1-methyl-(4-bromo-2,5-dioxo-2,5-dihydro-1H-3-pyrrolyl)-1H-2-indolyl)butyl)-1H-3-indolyl)-2,5-dihydro-1H-pyrrol-2,5-dion

Schmp.: 180 °C (Zers.)

### Beispiel 137

### 1-Methyl-3-(1-(4-(N-1,2-diaminoethyl))-4-(2-(4-(3-(1-methyl-(4-(N-1,2-diaminoethyl)-2,5-dioxo-2,5-dihydro-1H-3-pyrrolyl)-1H-2-indolyl)butyl)-1H-3-indolyl)-2,5-dihydro-1H-pyrrol-2,5-dion

Schmp.: > 240 °C (Zers.)

### Beispiel 138

### 4,39-Dimethyl-1,4,14,29,39,42-hexaazaoctacyclo[40.2.2.0(2,6).0(7,15).0(8,13).0(28,36). 0(30,35).0(37,41)]hexatetraconta-2(6),7(15),8(13),9,11,28(36),30(35),31,33,37(41)-decaen-3,5,38,40-tetraon

0.75 mmol 3-Bromo-4-(2-(12-(3-(4-bromo-1-methyl-2,5-dioxo-2,5-dihydro-1*H*-3-pyrrolyl)-1*H*-2-indolyl)dodecyl)-1*H*-3-indolyl)-1-methyl-2,5-dihydro-1*H*-pyrrol-2,5-dion werden in 200 ml absol. DMF gelöst, mit 0.5 ml abs. NEt₃ versetzt und auf 80 °C erwärmt. Anschließend tropft man zu der warmen Lösung langsam die Lösung von 0.75 mmol Piperazin in 100 ml absol. DMF und 0.5 ml NEt₃ und rührt danach 48 h bei 80 °C. Das Lösungsmittel wird dann weitestgehend *i*. *Vak.* entfernt und der Rückstand mit 100 ml 1N HCI versetzt. Diese Lösung wird dann mit Essigester (insgesamt ca. 600 ml) extrahiert, die vereinigten Extrakte werden über Na₂SO₄ getrocknet und das Lösungsmittel *i. Vak.* abgezogen. Die Reinigung erfolgt sc (SiO₂, CH₂Cl₂ / EE 9.5 : 0.5). Orange Kristalle, Ausb.: 0.267 g (52 %).
Schmp.: 194 - 195 °C

Analog wurden hergestellt:

### Beispiel 139

### 8,43-Dimethyl-5,8,18,33,43,46-hexaazanonacyclo[44.2.2.2(2,5).0(6,10).0(11,19).0(12,17).0(32,40).0(34,39).0(41,4 5)]dopentaconta-6(10),11 (19),12(17),13,15,32(40),34(39),35,37,41(45)-decaen-7,9,42,44-tetraon

Schmp.: > 250 °C

### Beispiel 140

### 9,44-Dimethyl-6,9,19,34,44,47-hexaazanonacyclo[45.2.2.2(3,6).0(7,11).0(12,20).0(15,18).0(33,412).0(35,40).0(42, 46)]tripentaconta-7(11),12(20),13(18),14,16,33(41),35(40),36,38,42(46)-decaen-8,10,43,45-tetraon

Schmp.: 286 °C (Zers.)

### Beispiel 141

### 10,45-Dimethyl-7,10,20,35,45,48-hexaazanonacyclo[46.2.2.2(4,7).0(8,12).0(13,21).0(14, 19).0(34,42).0(36,41).0(43,47)]tetrapentaconta-8(12),13(21),14(19),15,17,34(42),36(41), 37,39,43(47)-decaen-9,11,44,46-tetraon

Schmp.: > 250 °C

### Beispiel 142

### 11,46-Dimethyl-8,11,21,36,46,49-hexaazanonacyclo-[47.2.2.2(5,8).0(9,13).0(14,22).0(15,20).0(35,43).0(37,42).0(44,48)]pentapentaconta-9(13),14(22),15(20),16,18,35(43), 37(42),38, 40,44(48)-decaen-10,12,45,47-tetraon

Schmp.: 276 °C (Zers.)

### Beispiel 143

### 13,48-Dimethyl-10,13,23,38,48,51-hexaazanonacyclo[49.2.2.2(7,10).0(11,15).0(16,24). 0(17,22).0(37,45).0(39,44).0(46,50)]heptapentaconta-11(15),16(24),17(22),18,20,37(45),39(44),40,42,46(50)-decaen-12,14,47,49-tetraon

Schmp.: 245 °C (Zers.)

### Beispiel 144

### 14,49-Dimethyl-11,14,24,39,49,52-hexaazanonacyclo[50.2.2.2(8,11).0(12,16).0(17,25).0(18,23).0(38,46).0(40,45).0(4 7,51)]octapentaconta-12(16),17(25),18(23),19,21,38(46),40(45),41,43,47(51)-decaen-13,15,48,50-tetraon

Schmp.: 325 °C (Zers.)

### Beispiel 145

### 4,30-Dimethyl-1,4,14,20,30,33-hexaazaoctacyclo[31.2.2.0(2,6).0(7,15).0(8,13).0(19,27).0(21,26).0(28,32)]heptatria conta-2(6),7(15),8(13),9,11,19(27),21(26),22,24,28(32)-decaen-3,5,29,31-tetraon

Schmp.: 314 - 318 °C

### Beispiel 146

### 8,34-Dimethyl-5,8,18,24,34,37-hexaazanonacyclo[35.2.2.2(2, 5).0(6,10).0(11,19).0(12,17).0(23,31).0(25,30).0(32,3 6)]tritetraconta-6(10),11(19),12(17),13,15,23(31 ),25(30),26,28,32(36)-decaen-7,9,33,35-tetraon

Schmp.: 197 - 200 °C

### Beispiel 147

### 9,35-Dimethyl-6,9,19,25,35,38-hexaazanonacyclo[36.2.2.2(3,6).0(7,11).0(12,20).0(13,18).0(24,32).0(26,31).0 (33,37)]tetratetraconta-7(11),12(20),13(18),14,16,24(32),26(31 ),27,29,33(37)-decaen-8,10,34,36-tetraon

Schmp.: 337 °C (Zers.)

### Beispiel 148

### 10,36-Dimethyl-7,10,20,26,36,39-hexaazanonacyclo[37.2.2.2(4,7).0(8,12).0(13,21).0(14, 19).0(25,33).0(27,32).0(34, 38)]pentatetraconta-8(12),13(21),14(19),15,17,25(33),27(32),28, 30,34(38)-decaen-9,11,35,37-tetraon

Schmp.: 245 °C (Zers.)

### Beispiel 149

### 11,37-Dimethyl-8,11,21,27,37,40-hexaazanonacyclo[38.2.2.2(5,8).0(9,13).0(14,22).0(15, 20).0(26,34).0(28,33).0(35,39)]hexatetraconta-9(13),14(22),15(20),16,18,26(34),28(33),29, 31,35(39)-decaen-10,12,36,38-tetraon

Schmp.: 325 °C (Zers.)

### Beispiel 150

### 13,39-Dimethyl-10,13,23,29,39,42-hexaazanonacyclo[40.2.2.2(7,10).0(11,15).0(16,24). 0(17,22).0(28,36).0(30,35).0(37,41 )]octatetraconta-11(15),16(24),17(22),18,20,28(36), 30(35),31,33,37(41)-decaen-12,14,38,40-tetraon

Schmp.: 245 °C (Zers.)

### Beispiel 151

### 14,40-Dimethyl-11,14,24,30,40,43-hexaazanonacyclo[41.2.2.2(8,11).0(12,16).0(17, 25).0(18,23).0(29,37).0(31,36).0(38,42)]nonatetraconta-12(16),17(25), 18(23),19,21, 29(37),31(36),32, 34,38(42)-decaen-13;15,39,41-tetraon

Schmp.: 325 °C (Zers.)

### Beispiel 152

### 1,4,14,22,32,35-Hexaazaoctacyclo[33.2.2.0(2,6).0(7,15).0(8,13).0(21,29).0(23,28).0(30, 34)]nonatriaconta-2(6),7(15),8(13),9,11,21(29),23(28),24,26,30(34)-decaen-3,5,31,33-tetraon

Schmp.: 314 - 318 °C

### Beispiel 153

### 5,8,18,26,36,39-Hexaazanonacyclo[37.2.2.2(2,5).0(6,90).0(11,99).0(12,17).0(25,33).0(27, 32).0(34,38)]pentatetraconta-6(10),11(19),12(17),13,15,25(33),27(32),28,30,34(38)-decaen-7,9,35,37-tetraon

Schmp.: 197 - 200 °C

### Beispiel 154

### 9,37-Dimethyl-6,9,19,27,37,40-hexaazanonacyclo[38.2.2.2(3,6).0(7,11).0(12,20).0(13,18). 0(26,34).0(28,33).0(35,39)]hexatetraconta-7(11),12(20),13(18),14,16,26(34),28(33),29,31, 35(39)-decaen-8,10,36,38-tetraon

Schmp.: > 350 °C

### Beispiel 155

### 7,10,20,28,38,41-Hexaazanonacyclo[39.2.2.2(4,7).0(8,12).0(13,21).0(14,19).0(27, 35).0(29,34).0(36,40)]heptatetraconta-8(12),13(21),14(19),15,17,27(35),29(34),30,32, 36(40)-decaen-9,11,37,39-tetraon

Schmp.: 290 - 292 °C

### Beispiel 156

### 11,39-Dimethyl-8,11,21,29,39,42-hexaazanonacyclo[40.2.2.2(5,8).0(9,13).0(14,22). 0(15,20).0(28,36).0(30,35).0(37,41 )]octatetraconta-9(13),14(22),15(20),16,18,28(36),30(35),31,33,37(41)-decaen-10,12,38,40-tetraon

Schmp.: 310 °C (Zers.)

### Beispiel 157

### 13,41-Dimethyl-10,13,23,31,41,44-hexaazanonacyclo[42.2.2.2(7,10).0(11,15).0(16,24). 0(17,22).0(30,38).0(32,37).0(39,43)]pentaconta-11(15),16(24),17(22),18,20,30(38),32(37), 33,35,39(43)-decaen-12,14,40,42-tetraon

Schmp.: 310 °C (Zers.)

### Beispiel 158

### 14,42-Dimethyl-11,14,24,32,42,45-hexaazanonacyclo[43.2.2.2(8,11).0(12,16).0(17,25). 0(18,23).0(31,39). 0(33, 38).0(40,44)]unpentaconta-12(16),17(25),18(23),19,21,31(39), 33(38),34,36,40(44)-decaen-13,15,41,43-tetraon

Schmp.: 321 - 324 °C

### Beispiel 159

### 6,13-Dimethyl-5,6,7,8,9,10,11,12,13,14,19,20,21,22,23,24-hexadecahydrodipyrrolo [3',4':15,16:3',4':5,6]indolo[2',3':13,14][1,4]diazacyclohexadecino[8,7:b]indol-5,7,12,14-tetraon

Schmp.: > 240 °C

### Beispiel 160

### 1,4,14,29,39,42-Hexaazaoctacyclo[40.2.2.0(2,6).0(7,15).0(8,13).0(28,36).0(30,35).0 (37,41)]hexatetraconta-2(6),7(15),8(13),9,11,28(36),30(35),31,33,37(41)-decaen-3,5,38,40-tetraon

Schmp.: 194 - 195 °C

### Beispiel 161

### 5,8,18,33,43,46-Hexaazanonacyclo[44.2.2.2(2,5).0(6,10).0(11,19).0(12,17). 0(32,40).0(34,39).0(41,45)]dopentaconta-6(10),11(19),12(17),13,15,32(40),34(39),35,37,41 (45)-decaen-7,9,42,44-tetraon

Schmp.: 236 - 238 °C

### Beispiel 162

### 6,9,19,34,44,47-Hexaazanonacyclo[45.2.2.2(3,6).0(7,11).0(12,20).0(15,18).0 (33,412).0(35,40).0(42,46)]tripentaconta-(11),12(20),13(18),14,16,33(41),35(40), 36,38,42(46)-decaen-8,10,43,45-tetraon

Schmp.: 231 - 233 °C

### Beispiel 163

### 7,10,20,35,45,48-Hexaazanonacyclo[46.2.2.2(4,7).0(8,12).0(13,21).0(14, 19).0(34,42).0(36,41).0(43,47)]tetrapentaconta-8(12),13(21),14(19),15,17,34(42),36(41),37,39,43(47)-decaen-9,11,44,46-tetraon

Schmp.: 209-211 °C

### Beispiel 164

### 8,11,21,36,46,49-Hexaazanonacyclo[47.2.2.2(5,8).0(9,13).0(14,22).0(15,20) .0(35,43).0(37,42).0(44,48)]pentapentaconta-9(13),14(22),15(20),16,18,35(43), 37(42),38,40,44(48)-decaen-10,12,45,47-tetraon

Schmp.: 282 - 284 °C

### Beispiel 165

### 10,13,23,38,48,51-Hexaazanonacyclo[49.2.2.2(7,10).0(11,15).0(16,24). 0(17,22).0(37,45).0(39,44).0(46,50)]heptapentaconta-11(15),16(24),17(22),18,20,37(45),39(44),40,42,46(50)-decaen-12,14,47,49-tetraon

Schmp.: 176 - 179 °C

### Beispiel 166

### 11,14,24,39,49,52-Hexaazanonacyclo[50.2.2.2(8,11).0(12,16).0(17,25).0(18 ,23).0(38,46).0(40,45).0(47,51 )]octapentaconta-12(16),17(25),18(23),19,21,38(46),40(45), 41,43,47(51)-decaen-13,15,48,50-tetraon

Schmp.: 147 - 150 °C

### Beispiel 167

### 1,4,14,20,30,33-Hexaazaoctacyclo[31.2.2.0(2,6).0(7,15).0(8,13).0(19,27).0 (21,26).0(28,32)]heptatriaconta-2(6),7(15),8(13),9,11,19(27),21(26),22,24,28(32)-decaen-3,5,29,31-tetraon

Schmp.: 350 °C (Zers.)

### Beispiel 168

### 5,8,18,24,34,37-Hexaazanonacyclo[35.2.2.2(2,5).0(6,10).0(11,19).0(12,17).0 (23,31).0(25,30).0(32,36)]tritetraconta-6(10),11(19),12(17),13,15,23(31),25(30),26,28,32 (36)-decaen-7,9,33,35-tetraon

Schmp.: 285 °C (Zers.)

### Beispiel 169

### 6,9,19,25,35,38-Hexaazanonacyclo[36.2.2.2(3,6).0(7,11).0(12,20).0(13,18).0 (24,32).0(26,31).0(33,37)]tetratetraconta-7(11),12(20),13(18),14,16,24(32),26(31 ),27,29,33(37)-decaen-8,10,34,36-tetraon

Schmp.: 215 °C

### Beispiel 170

### 7,10,20,26,36,39-Hexaazanonacyclo[37.2.2.2(4,7).0(8,12).0(13,21).0(14, 19).0(25,33).0(27,32).0(34,38)]pentatetraconta-8(12),13(21),14(19),15,17,25(33),27(32),28, 30,34(38)-decaen-9,11,35,37-tetraon

Schmp.: 330 °C (Zers.)

### Beispiel 171

### 8,11,21,27,37,40-Hexaazanonacyclo[38.2.2.2(5,8).0(9,13).0(14,22).0(15, 20).0(26,34).0(28,33).0(35,39)]hexatetraconta-9(13),14(22),15(20),16,18,26(34),28(33),29, 31,35(39)-decaen-10,12,36,38-tetraon

Schmp.: 335.5 °C (Zers.)

### Beispiel 172

### 10,13,23,29,39,42-Hexaazanonacyclo[40.2.2.2(7,10).0(11,15).0(16,24). 0(17,22).0(28,36).0(30,35).0(37,41)]octatetraconta-11(15),16(24),17(22),18,20,28(36), 30(35),31,33,37(41)-decaen-12,14,38,40-tetraon

Schmp.: 243 - 245 °C

### Beispiel 173

### 11,14,24,30,40,43-Hexaazanonacyclo[41.2.2.2(8,11).0(12,16).0(17, 25).0(18,23).0(29,37).0(31,36).0(38,42)]nonatetraconta-12(16),17(25),18(23),19,21, 29(37),31(36),32,34,38(42)-decaen-13,15,39,41-tetraon

Schmp.: 258 - 260 °C

### Beispiel 174

### 4,32-Dimethyl-1,4,14,22,32,35-hexaazaoctacyclo[33.2.2.0(2,6).0(7,15).0(8,13).0(21,29).0(23,28).0(30,34)]nonatria conta-2(6),7(15),8(13),9,11,21(29),23(28),24,26,30(34)-decaen-3,5,31,33-tetraon

Schmp.: > 350 °C

### Beispiel 175

### 8,36-Dimethyl-5,8,18,26,36,39-hexaazanonacyclo[37.2.2.2(2,5).0(6,10).0(11,19).0(12,17).0(25,33).0(27,32).0(34,3 8)]pentatetraconta-6(10),11(19),12(17),13,15,25(33),27(32),28,30,34(38)-decaen-7,9,35,37-tetraon

Schmp.: 310 °C (Zers.)

### Beispiel 176

### 10,38-Dimethyl-7,10,20,28,38,41-hexaazanonacyclo[39.2.2.2(4,7).0(8,12).0(13,21).0(14,19).0(27,35).0(29,34).0(36,4 0)]heptatetraconta-8(12),13(21),14(19),15,17,27(35),29(34),30,32,36(40)-decaen-9,11,37,39-tetraon

Schmp.: 280 °C (Zers.)

### Beispiel 177

### 13,46-Dimethyl-1,7,10,13,23,36,46,49-octaazanonacyclo[47.2.2.2(7,10).0(11,15).0(16,24).0(17,22).0(35,43).0(37,42).0(44 ,48)]pentapentaconta-11(15),16(24),17(22),18,20,35(43),37(42),38,40,44(48)-decaen-12,14,45,47-tetraon

Schmp.: > 220 °C

### Beispiel 178

### 4,31-Dimethyl-1,4,14,21,31,34-hexaazaoctacyclo[32.2.2.0(2,6).0(7,15).0(8,13).0(20,28).0(22,27).0(29,33)]octatriaconta-2(6),7(15),8(13),9,11,20(28),22(27),23,25,29(33)-decaen-3,5,309,32-tetraon

Schmp.: > 240 °C (Zers.)

### Beispiel 179

### 8,35-Dimethyl-5,8,18,25,35,38-hexaazanonacyclo[36.2.2.2(2,5).0(6,10).0(11,19).0(12,17).0(24,32).0(26,31).0(33,3 7)]tetratetraconta-6(10),11(19),12(17),13,15,24(32),26(31),27,29,33(37)-decaen-7,9,34,36-tetraon, Schmp.: > 240 (Zers.)

### Beispiel 180

### (1-(2-Dimethylaminoethyl)-1H-3-indolyl)(1H-3-indolyl)-1-methanon

Man löst 0.5 g Bis(indol-3-yl)methanon in 30 ml Aceton. Nach Zugabe von 0.92 g K₂CO₃ und 0.27 g 2-Dimethylamino-1-chlorethan-hydrochlorid wird 70 h zum Rückfluß erhitzt. Das Aceton wird abgezogen und der Rückstand mit 30 ml Wasser und 30 ml Essigester versetzt. Nach 15 min Rühren trennt man die org. Phase ab und schüttelt die Wasserphase noch zwei mal mit je 15 ml Essigester aus. Die vereinigten org. Phasen werden über Na₂SO₄ getrocknet und das Lösungsmittel abgezogen. Die Reinigung erfolgt sc (SiO₂, EE / MeOH 10:1). Ausb.: 0.14 g (20 %)
Smp.: 180 - 182 °C

Analog wurden hergestellt:

### Beispiel 181

### (1-(2-Morpholinoethyl)-1H-3-indolyl)(1H-3-indolyl)-1-methanon

Smp.: 192 - 194 °C

### Beispiel 182

### Bis(1-(2-morpholinoethyl)-1H-3-indolyl)-1-methanon

Smp.: 91 - 93 °C

### Beispiel 183

### (1-(2-Piperidinoethyl)-1H-3-indolyl)(1H-3-indolyl)-1-methanon

Smp.: 223 - 225 °C

### Beispiel 184

### Bis(1-(2-piperidinoethyl)-1H-3-indolyl)-1-methanon

Smp.: 152 - 155 °C

### Beispiel 185

### (1-(3-Dimethylaminopropyl)-1H-3-indolyl)(1H-3-indolyl)-1-methanon

Smp.: 144-146 °C

### Beispiel 186

### (1-(3-Pyrrolidinopropyl)-1H-3-indolyl)(1H-3-indolyl)-1-methanon

Smp.: 148-152 °C

### Beispiel 187

### (1-(2-Dimethylaminoethyl)-1H-2-indolyl)(1H-2-indolyl)-1-methanon

Smp.: 147-150°C

### Beispiel 188

### (1-(2-Morpholinoethyl)-1H-2-indolyl)(1H-2-indolyl)-1-methanon

Wachs

### Beispiel 189

### (1-(2-Piperidinoethyl)-1H-2-indolyl)(1H-2-indolyl)-1-methanon

Wachs

### Beispiel 190

### (1-(2-Pyrrolidinoethyl)-1H-2-indolyl)(1H-2-indolyl)-1-methanon

Wachs

### Beispiel 191

### 11,46-Dimethyl-21,36-bis(2-(1-piperindinyl)-ethyl)-8,11,21,36,46,49-hexaazanonacyclo-[47.2.2.2(5,8).0(9,13).0(14,22).0(15,20).0(35,43).0(37,42).0(44,48)]pentapentaconta-9(13),14(22),15(20),16,18,35(43), 37(42),38, 40,44(48)-decaen-10,12,45,47-tetraon

Schmp.: 125 - 130 °C

### Beispiel 192

### 3,3'-Dimethoxydiglyoxyl-1,8-(2,2'-bisindolyl)octan

Unter N₂-Atmosphäre tropft man zu einer Lösung von 1.15 g (4.00 mmol) 1,8-(2,2'bisindolyl)octan in 20 ml absol. THF bei 0 °C Oxalyldichlorid zu und rührt 2 h bei Raumtemperatur. Anschließend läßt man 20 ml MeOH zutropfen. Der Ansatz wird über Nacht bei Raumtemperatur gerührt. Zur Aufarbeitung versetzt man den Ansatz mit 100 ml 1 N HCl, neutralisiert mit 2 N NaOH und extrahiert die Mischung mit EE (3 x 25 ml). Nach Trocknen über NaSO₄ wird das Lösungsmittel abgezogen.
Schmp.: > 250 °C (Zers.)

### Beispiel 193

### 3-(2-(4-(1H-2-indolyl)butyl)-1H-3-indolyl)-1-methyl-2,5-pyrrolidindion

Unter H₂-Atmosphäre rührt man eine Lösung von 240 mg (0.50 mmol) 3-Bromo-4-(2-(4-(1*H*-2-indolyl)butyl)-1*H*-3-indolyl)-1-methyl-2,5-dihydro-1*H*-pyrrol-2,5-dion und 140 mg (0.25 mmol) Pd(OH)₂/C (20 %) in 30 ml MeOH 24 h bei Raumtemperatur. Zur Aufarbeitung filtriert man den Ansatz, engt das Filtrat ein und reinigt den Rückstand sc (SiO₂; CH₂Cl₂/EE 95 : 5). Beim Einengen der Reinfraktion wird das Produkt durch Zugabe von PE zur Kristallisation gebracht.Ausb.: 48.0 mg (24 %), beiges Pulver
Schmp.: 180 - 182 °C

### Beispiel 194

### Test zur Messung der Hemmung der PDGF-abhängigen Tyrosinphosphorylierung für erfindungsgemäße Verbindungen

Swiss 3T3-Zellen werden für 1 Woche unter Standardbedingungen (DMEM mit Glutamin, 4g Glukose/l, 10% FKS Antibiotika, 5-7,5 % CO₂) kultiviert und sind am Ende der Kulturperiode konfluent und nicht mehr proliferierend. Das Medium wird durch serumfreies DMEM ersetzt und die Zellen werden mit den erfindungsgemäßen Verbindungen oder in Kontrollversuchen mit DMSO (Endkonzentration 0,1-1%) für 2 h bei 37°C inkubiert. Die Zellen werden dann durch Zugabe von PDGF-BB zu einer Endkonzentration von 100 ng/ml für 5 min bei Raumtemperatur stimuliert, in Kontrollen erfolgt Zugabe des entsprechenden Lösemittels. Dann erfolgt zweimaliges Waschen der Zellen mit eiskalter PBS und Lyse der Zellen in einem Triton X-100-haltigen Lysispuffer (Zusammensetzung und Verfahren wie in Selective plateletderived growth factor receptor kinase blockers reverse sis-transformation M. Kovalenko, A. Gazit, A. Böhmer, C. Rorsman, L. Rönnstrand, C.H. Heldin, J. Waltenberger, F.D. Böhmer, A. Levitzki (1994) Cancer Res. 54, 6106-6114 beschrieben). Die Lysate werden zentrifugiert und die Eiweißkonzentration wird bestimmt. 10µg Lysatprotein werden direkt auf Nitrozellulose-Membranen aufgetragen (Dot-Blot-Apparatur oder entsprechende Multiwellplatten mit Nitrozellulose-Boden).

Der Nachweis der Tyrosinphosphorylierung erfolgt mit Antiphosphotyrosin-Antikörpern nach Standardverfahren. Typischerweise wird ein monoklonaler Antiphosphotyrosin-Antikörper, konjugiert mit Meerrettich-Peroxidase (POD) und Detektion der POD-Aktivität mittels Chemiluminiszenz-Nachweis verwendet. Die Quantifizierung erfolgt entweder über Grauwertanalysen von zur Luminiszenz-Detektion verwendeten Filmen oder direkt mit einem Luminometer. Üblicherweise resultiert die PDGF-Stimulation der Zellen in einer 3-10-fachen Verstärkung des Signals.

Die Verbindungen wurden primär zweifach in der Endkonzentration 10 µg/ml eingesetzt. Bei aktiven Verbindungen erfolgte eine Titration in den Stufen 30 µM, 10 µM, 3 µM, 1 µM, 0,3 µM und 0,1 µM als Doppelbestimmung. Die Ergebnisse sind in der Tabelle 1 gezeigt.

**Tabelle 1**

| Beispiel | Verbindung | IC 50 (µM) |
|---|---|---|
| 19 | Bisindol-2-ylmethan-1-on | 1 |
| 20 | (5-Methoxyindol-2-yl)-(indol-2-yl)methan-1-on | 0,1 - 0,3 |
| 21 | Bis(5-methoxyindol-2-yl)-1-methanon | 10 - 30 |
| 28 | Benzo[*b*]thiophen-2-yl(5-methoxy-1*H*-2-indolyl)-1-methanon | 1 |
| 43 | 5-Hydroxy-1*H*-2-indolyl(1*H*-2-indolyl)methanon | 0,1 - 0,3 |
| 45 | 1*H*-2-Indolyl[5-(2-morpholin-1-ylethyloxy)-1*H*-2-indolyl]methanon | 1 - 3 |
| 48 | 1*H*-2-Indolyl[5-(2-dimethylaminoethyloxy)-1*H*-2-indolyl]methanon | 0,3 - 1 |
| 53 | [2-(1*H*-2-lndolylcarbonyl)-1*H*-5-indolyl)] ethanoat | 0; 1-0,3 |
| 55 | [2-(1*H*-2-Indolylcarbonyl)-1*H*-5-indolyl)] butanoat | 1 - 3 |
| 56 | [2-(1*H*-2-Indolylcarbonyl)-1*H*-5-indolyl)] 2-(*N,N*)-dimethylaminoethanoat | 0,1 |
| 57 | [2-(1*H*-2-Indolylcarbonyl)-1*H*-5-indolyl)] propanoat | 0,3 - 1 |
| 58 | [2-(1*H*-2-Indolylcarbonyl)-1*H*-5-indolyl)]2-thiophenylethanoat | 0,3 - 1 |

Der qualitative Nachweis der Effekte auf die Tyrosinphosphorylierung des PDGF-Rezeptors und zellulärer Substrate erfolgt durch Analyse der Zell-Lysate mittels Polyakrylamidgelelktrophorese und Immunoblotting mit Anti-Phosphotyrosinantikörpem nach Standardverfahren.

Weiterhin wurden die erfindungsgemäßen Verbindungen in vitro mit isolierten Plasmamembranen von Swiss 3T3 Zellen und mit aus überexprimierenden Zellen gereinigtem PDGF-Rezeptor untersucht, in intakten A431-Zellen (und teilweise auch in Swiss 3T3 Plasmamembranen) auf eine mögliche Hemmung der EGF Rezeptortyrosinkinase getestet und auf Hemmung rekombinanter Src-Kinase getestet. Die Ergebnisse sind in Tabelle 2 gezeigt.

DNA-Synthese-Tests in Swiss 3T3-Zellen, die mit unterschiedlichen Wachstumsfaktoren stimuliert werden, sind geeignet, selektive antiproliferative Wirkungen von Rezeptor-Tyrosinkinasehemmstoffen zu charakterisieren. Die Verbindungen wurden hinsichtlich ihrer Wirkung auf die durch PDGF-BB, bFGF, FCS und die Kombination von EGF und Insulin in diesen Zellen stimulierte DNA-Synthese untersucht. Diese Stimulantien sind annähernd equipotent und erhöhen die DNA-Synthese in vorher Wachstums-arretierten Swiss 3T3-Zellen auf das 5-20-fache. Die Dosis-Abhängigkeiten der ensprechenden Versuche sowie die erhaltenen IC50-Werte sind ebenfalls in Tabelle 2 dargestellt.

Weiterhin wurden die Verbindungen auf eine mögliche anti-transformierende Wirkung unter Verwendung von *sis*-transformierten NIH3T3-Zellen untersucht. In diesen Zellen wird ein u.a. durch irreguläres mehrschichtiges Wachstum und Koloniebildung in Weichagar gekennzeichneter transformierter Phänotyp durch Expression von PDGF-BB und permanente Aktivierung der endogenen PDGF-Rezeptoren aufrecht erhalten. Die erhaltenen IC50-Werte sind ebenfalls in Tabelle 2 dargestellt.

Demnach wurden Wirkungen auf die PDGF-Rezeptorkinase durch die Verbindungen in folgenden Tests gefunden:
- PDGF-Rezeptor-Autophosphorylierung in intakten Swiss 3T3-Zellen,
- PDGF-Rezeptor-Autophosphorylierung in isolierten Membranen von Swiss 3T3-Fibroblasten und
- PDGF-Rezeptor-Autophosphorylierung in gereinigten Rezeptorpräparaten. Keine Wirkungen wurden in analogen Tests mit der Rezeptor-Tyrosinkinase für den Epidermalen Wachstumsfaktor sowie mit der cytosolischen Tyrosinkinase Src bis zu einer Konzentration von 30 µM beobachtet. Damit weisen die Verbindungen Spezifität für die Hemmung der PDGF-Rezptor-Tyrosinkinase gegenüber anderen Tyrosinkinasen auf.

**Tabelle 2**

| Test | | IC 50 (µM) | | |
|---|---|---|---|---|
| | | Beispiel 19 | Beispiel 20 | Beispiel 21 |
| PDGFR-Phosphorylierung in vivo (Swiss 3T3-Zellen) | | 1 | 0,1 -0,3 | 10 - 30 |
| PDGFR-Phosphorylierung in vitro (Swiss 3T3-Membrane) | | 0,3 - 1 | < 0,03 | n.d. |
| PDGFR-Phosphorylierung in vitro (gereinigter PDGF-Rezeptor) | | 0,1 - 0,3 | n.d. | n.d. |
| EGFR-Phosphorylierung in vivo (A 431-Zellen) | | > 10 | > 10 | n.d. |
| src-Kinase-Phosphorylierung in vivo (src-NIH-Zellen) | | > 30 | > 30 | n.d. |
| Reversion der transformierten Morphologie von sis-3T3-Zellen | | +++ | n.d. | n.d. |
| DNA-Synthese (Swiss 3T3-Zellen) | PDGF-stimuliert | 3 - 10 | n.d. | n.d. |
| | FGF-stimuliert | 3 - 10 | n.d. | n.d. |
| | EGF/Insulin-stimuliert | > 30 | n.d. | n.d. |
| | 10 % FCS | > 30 | n.d. | n.d. |
| Koloniebildung (sis-3T3-Zellen) | | 3 - 10 | n.d. | n.d. |

## Patentansprüche

1. Verbindungen der allgemeinen Formel I: 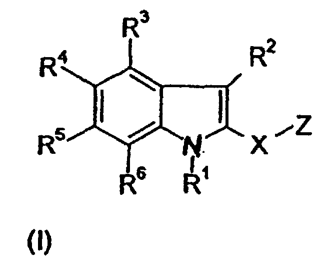 worin Z eine Gruppe mit der allgemeinen Formel (II) ist, 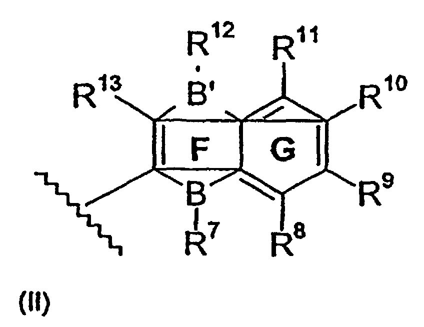 wobei B, B' Kohlenstoff, Stickstoff-, Sauerstoff- und Schwefelatom sein kann und die Ringsysteme F und G unabhängig voneinander sowohl gesättigte als auch ungesättigte 5- und 6-Ringe sein können,
X eine Gruppe mit der allgemeinen Formel III oder IV darstellt,
―(CH₂)ₗ―[CR¹⁴R¹⁵]ₘ―(CH₂)ₙ (III) 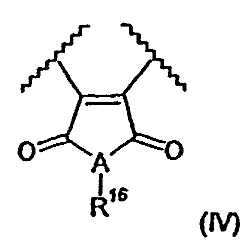
worin A, ein N-, O-, S- Atom sein kann, I und n die Zahlen von 0 bis 6, m die Zahlen 1 und 2 einnehmen können, sowie R¹⁴ und R¹⁵ entweder zusammen ein Sauerstoffatom bilden oder R¹⁴ eine Hydroxylgruppe und R¹⁵ ein Wasserstoffatom bedeuten oder R¹⁴ und R¹⁵ Wasserstoffatome bedeuten und wobei R¹⁶ ein Wasserstoffatom, ein Alkyl- oder Arylrest, halogen-, amino, oder azidosubstituierter Alkyl- oder Arylrest, ein Alkyloxymethylrest oder substituierter Alkyloxymethylrest bedeutet,
R² und R¹³ zusammen eine Verknüpfung mit der allgemeinen Formel V oder VI bilden 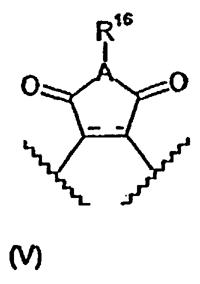 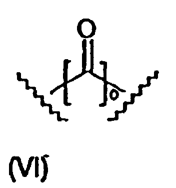 wobei die gestrichelte Bindung eine Doppel- oder Einfachbindung bedeutet, A und R¹⁶ die selbe Bedeutung wie oben besitzen und o die Zahlen 1 und 2 annehmen kann,
R² und R¹³ gleiche oder verschieden Reste der allgemeinen Formel VII oder Wasserstoffatome bedeuten, 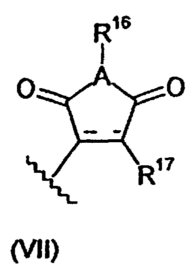 wobei die gestrichelte Bindung eine Doppel- oder Einfachbindung bedeutet, A und R¹⁶ die selbe Bedeutung wie oben besitzen und R¹⁷ ein Halogenatom oder einen Rest der allgemeinen Formel VIII bedeutet, 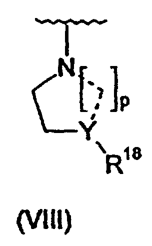 so daß p = 0, 1 oder 2 sein kann (wenn p = 0 dann handelt es sich um ein acyclisches primäres Amin und Y trägt ein zusätzliches Wasserstoffatom), Y ein Kohlenstoff, Sauerstoff- oder Stickstoffatom sein kann und wenn Y ein Kohlenstoff- oder Stickstoffatom ist R¹⁸ ein Wasserstoffatom oder einen Alkyl- oder Arylrest, substituierten Alkyl- oder Arylrest, gesättigten oder ungesättigten Heterozyklus, Alkoxycarbonylrest, Aminocarbonylmethylrest, substituierten Aminocarbonylmethylrest bedeutet,
R²und R¹³ zusammen eine Verknüpfung mit der allgemeinen Formel IX oder X bilden 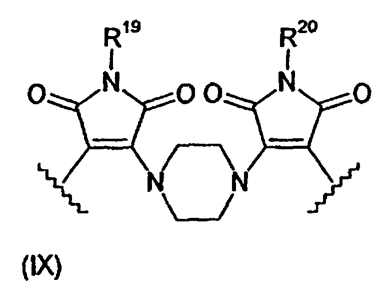 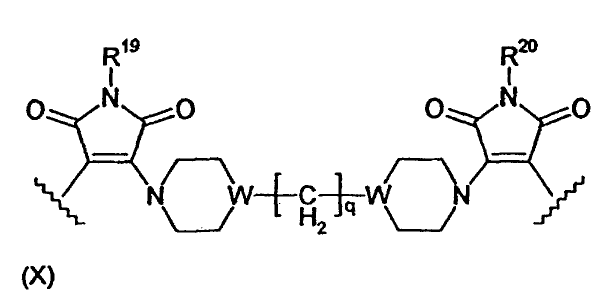 wobei W entweder ein Kohlenstoff- oder ein Stickstoffatom darstellt, q eine Zahl zwischen 0 und 6 einnehmen kann und R¹⁹ und R²⁰ Wasserstoffatome, Alkyl- oder substituierte Alkylreste bedeuten kann,
worin R¹, R⁷ und R¹² gleich oder verschieden sind und Wasserstoffatome, Alkyl- und Aminoalkylreste, Phenylsutphonylreste, Alkylsilylmethoxymethylreste, einen Zucker oder substituierter Zucker bedeuten,
wobei R³, R⁴, R⁵, R⁶, R⁸, R⁹, R¹⁰ und R¹¹ gleich oder verschieden sind und jeweils ein Wasserstoffatom, eine alkoxy-, amino-, halogen-, cycloalkyl-, cycloheteroalkyl-, aryl- oder heteroarylsubstituierte Alkyl-, Alkoxy, Alkoxymethylgruppe, Nitrogruppe, ein Halogenatom oder eine O-Alkoxygruppe der allgemeinen Form -O-(C=O)-R²¹ darstellt, wobei R²¹ eine alkoxy-, amino-, halogen-, cycloalkyl-, cydoheteroalkyl-, aryl- oder heteroarylsubstituierte Alkyl-, Alkoxy- oder Alkoxyntethylgruppe bedeutet.

2. Verbindungen nach Anspruch 1 mit der allgemeinen Formel XI 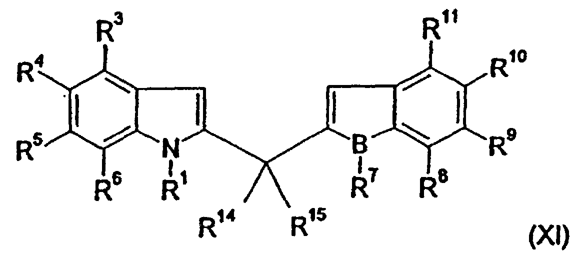 worin B ein Stickstoff-, Sauerstoff- oder Schwefelatom bedeuten und R³, R⁴, R⁵, R⁶, R⁷, R⁸, R⁹, R¹⁰, R¹¹, R¹⁴ und R¹⁵ dieselbe Bedeutung wie oben haben.

3. Verbindungen nach Anspruch 1 mit der allgemeinen Formel I worin X eine Gruppe mit der allgemeinen Formel III oder IV nach Anspruch 1 darstellt und R¹ und R² Wasserstoffatome bedeuten, A und B Stickstoffatome bedeuten sowie R¹, R³, R⁴, R⁵, R⁶, R⁷, R⁸, R⁹, R¹⁰, R¹¹ und R¹⁶ dieselbe Bedeutung wie oben haben.

4. Verbindungen nach Anspruch 1 mit einer der allgemeinen Formeln XIII und XIV 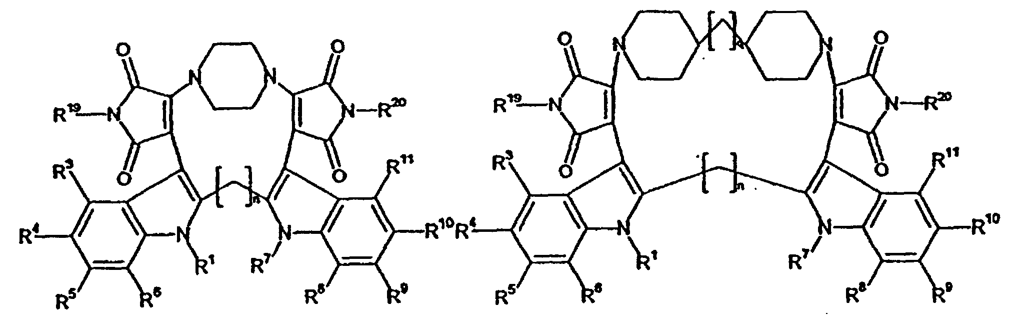 worin n die Zahlen 3, 4, 5, 8, 12, q die Zahlen 0, 1, 2, 3, 5, 6 bedeutet, R¹⁹, R²⁰ Wasserstoffatome oder Alkylgruppen bedeuten sowie R¹, R³, R⁴, R⁵, R⁶, R⁷, R⁸, R⁹, R¹⁰, R¹¹ und R¹⁶ gleich oder verschieden sind und dieselbe Bedeutung wie oben besitzen.

5. Verbindungen nach Anspruch 1 mit der allgemeinen Formel XV 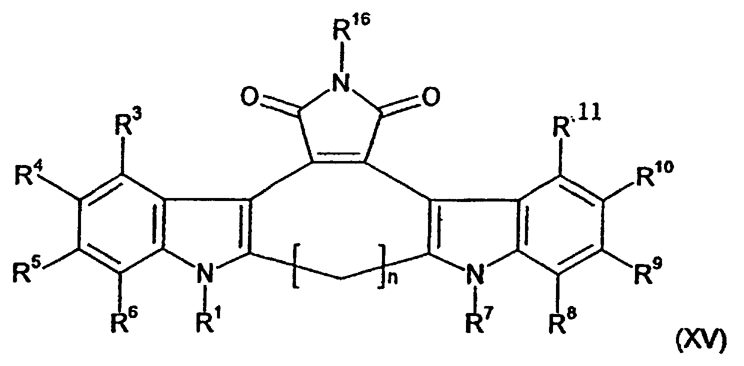 worin n die Zahlen 1, 2, 3 bedeutet, R¹⁶ ein Wasserstoffatom oder eine Alkylgruppe bedeutet sowie R¹, R³, R⁴, R⁵, R⁶, R⁷, R⁸, R⁹, R¹⁰, R¹¹ und R¹⁶ gleich oder verschieden sind und dieselbe Bedeutung wie oben besitzen.

6. Bisindol-2-ylmethan-1-on nach Anspruch 1.

7. (5-Methoxyindol-2-yl)-(indol-2-yl)methan-1-on nach Anspruch 1.

8. Bis(5-methoxyindol-2-yl)-1-methanon nach Anspruch 1.

9. Benzo[*b*]thiophen-2-yl(5-methoxy-1*H*-2-indolyl)-1-methanon nach Anspruch 1.

10. 5-Hydroxy-1*H*-2-indolyl(1*H*-2-indolyl)methanon nach Anspruch 1.

11. 1*H*-2-Indolyl[5-(2-morpholin-1-ylethyloxy)-1*H*-2-indolyl]methanon nach Anspruch 1.

12. 1*H*-2-Indolyl[5-(2-dimethylaminoethyloxy)-1*H*-2-indolyl]methanon nach Anspruch 1.

13. [2-(1*H*-2-lndolylcarbonyl)-1*H*-5-indolyl)] ethanoat nach Anspruch 1.

14. [2-(1*H*-2-Indolylcarbonyl)-1*H*-5-indolyl)] butanoat nach Anspruch 1.

15. [2-(1*H*-2-Indolylcarbonyl]-1*H*-5-indolyl)]2-(*N*,*N*)-dimethylaminoethanoat nach Anspruch 1.

16. [2-(1*H*-2-Indolylcarbonyl)-1*H*-5-indolyl)] propanoat nach Anspruch 1.

17. [2-(1*H*-2-Indolylcarbonyl)-1*H*-5-indolyl)] 2-thiophenylethanoat nach Anspruch 1.

18. Arzneimittel, umfassend eine Verbindung nach einem der Ansprüche 1 bis 17.

19. Verbindung der Formel I nach einem der Ansprüche 1 bis 17 zur Verwendung als ein Tyrosinkinase inhibierendes Mittel.

20. Verbindung der Formel I nach einem der Ansprüche 1 bis 17 zur Verwendung als ein PDGF-Rezeptor-Tyrosinkinase oder eine strukturell verwandte Rezeptor-Tyrosinkinase inhibierendes Mittel.

21. Verbindung der Formel I nach einem der Ansprüche 1 bis 17 zur Verwendung bei der Behandlung von Tumoren.

22. Verbindung der Formel I nach einem der Ansprüche 1 bis 17 zur Verwendung bei der Behandlung von Arteriosklerose, Restenose nach Ballon-Angioplasie, Arthritis und fibrotischen Erkrankungen.

23. Verfahren zur Herstellung von Verbindungen nach Anspruch 1, bei denen B ein N-Atom, R² und R¹³ einen Rest mit der allgemeinen Formel V nach Anspruch 1 bedeuten oder zusammen eine Verknüpfung mit der allgemeinen Formel VII nach Anspruch 1 bilden, **dadurch gekennzeichnet, daß** man ein 2,2'-Bis-1*H*-Indolylalkan oder ein Derivat desselben mit der allgemeinen Formel XI 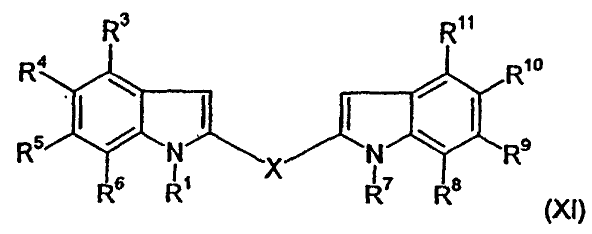 in der X, R¹, R³, R⁴, R⁵, R⁶, R⁷, R⁸, R⁹, R¹⁰ und R¹¹ die gleiche Bedeutung wie oben besitzen, mit Dibrommaleinimid umsetzt.

24. Verfahren zur Herstellung von Verbindungen nach Anspruch 1, bei denen B ein N-Atom, R² und R¹³ zusammen eine Verknüpfung mit der allgemeinen Formel IX oder X nach Anspruch 1 bilden, **dadurch gekennzeichnet, daß** man zunächst eine 2,2'-Bis-1*H*-Indolylalkan oder ein Derivat desselben mit der allgemeinen Formel XI 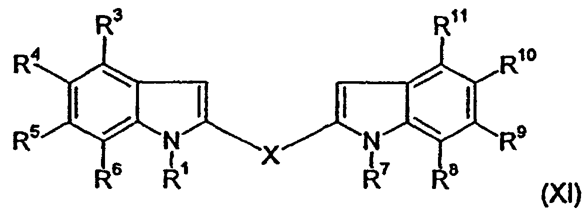 in der X, R¹, R³, R⁴, R⁵, R⁶, R⁷, R⁸, R⁹, R¹⁰ und R¹¹ die gleiche Bedeutung wie oben besitzen, mit Dibrommaleinimid umsetzt und anschließend mit einem primären oder sekundären Amin folgender allgemeiner Strukturen XVI, XVII oder Piperazin 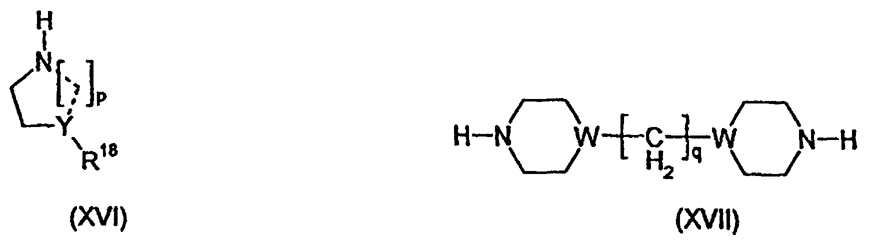 worin p, q, R¹⁷ und W die selbe Bedeutung wie oben besitzen, umsetzt.

## Claims

1. Compounds of the general formula I: 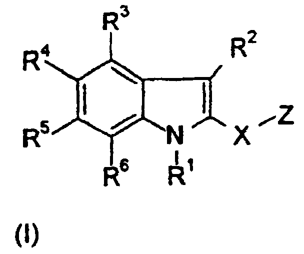 in which Z is a group having the general formula (II) 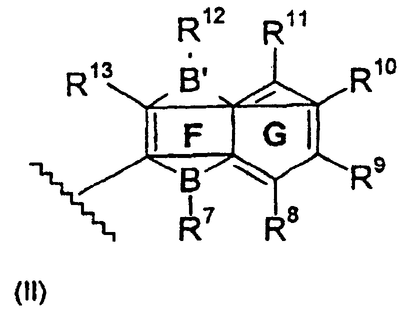 where B, B' can be a carbon, nitrogen, oxygen or sulphur atom and the ring systems F and G independently of one another can be either saturated or unsaturated 5- and 6-membered rings, X is a group having the general formula III or IV
―(CH₂)ₗ―[CR¹⁴R¹⁵]ₘ―(CH₂)ₙ (III)
in which A can be an N, O or S atom, 1 and n can assume the numbers from 0 to 6, m the numbers 1 and 2, and R¹⁴ and R¹⁵ either together form an oxygen atom or R¹⁴ is a hydroxyl group and R¹⁵ is a hydrogen atom or R¹⁴ and R¹⁵ are hydrogen atoms and where R¹⁶ is a hydrogen atom, an alkyl or aryl radical, halogen-, amino-, or azido-substituted alkyl or aryl radical, an alkyloxymethyl radical or substituted alkyloxymethyl radical,
R² and R¹³ together form a linkage having the general formula V or VI 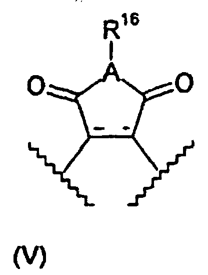 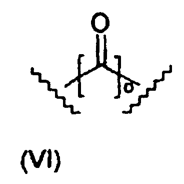 where the dashed bond is a double or single bond, A and R¹⁶ have the same meaning as above and o can assume the numbers 1 and 2,
R² and R¹³ are identical or different radicals of the general formula VII or hydrogen atoms, 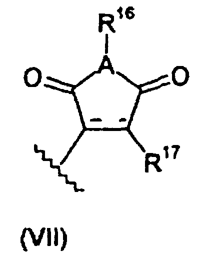 where the dashed bond is a double or single bond, A and R¹⁶ have the same meaning as above and R¹⁷ is a halogen atom or a radical of the general formula VIII 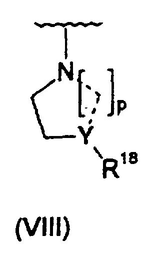 such that p can be = 0, 1 or 2 (if p = 0 then it is an acyclic primary amine and Y carries an additional hydrogen atom), Y can be a carbon, oxygen or nitrogen atom, and if Y is a carbon or nitrogen atom, R¹⁸ is a hydrogen atom or an alkyl or aryl radical, substituted alkyl or aryl radical, saturated or unsaturated heterocycle, alkoxycarbonyl radical, aminocarbonylmethyl radical or substituted aminocarbonylmethyl radical,
R² and R¹³ together form a linkage having the general formula IX or X 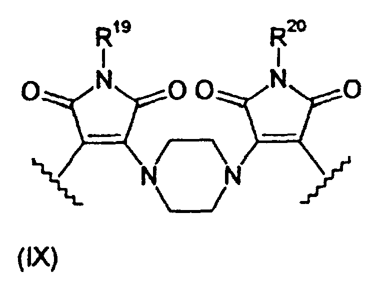 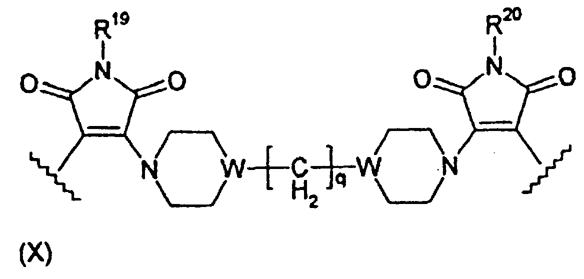 where W is either a carbon or a nitrogen atom, q can assume a number between 0 and 6 and R¹⁹ and R²⁰ can be hydrogen atoms, alkyl radicals or substituted alkyl radicals,
in which R¹, R⁷ and R¹² are identical or different and are hydrogen atoms, alkyl or aminoalkyl radicals, phenylsulphonyl radicals, alkylsilylmethoxymethyl radicals, a sugar or substituted sugar,
where R³, R⁴, R⁵, R⁶, R⁸, R⁹, R¹⁰ and R¹¹ are identical or different and in each case is a hydrogen atom, an alkoxy-, amino-, halogen-, cycloalkyl-, cycloheteroalkyl-, aryl- or heteroaryl-substituted alkyl, alkoxy or alkoxymethyl group, nitro group, a halogen atom or an O-alkoxy group of the general form -O-(C=O)-R²¹, where R²¹ is an alkoxy-, amino-, halogen-, cycloalkyl-, cycloheteroalkyl-, aryl- or heteroaryl-substituted alkyl, alkoxy or alkoxymethyl group.

2. Compounds according to Claim 1 having the general formula XI 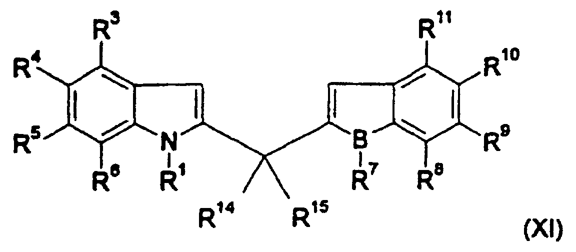 in which B is a nitrogen, oxygen or sulphur atom and R³, R⁴, R⁵, R⁶, R⁷, R⁸, R⁹, R¹⁰, R¹¹, R¹⁴ and R¹⁵ have the same meaning as above.

3. Compounds according to Claim 1 having the general formula I in which X is a group having the general formula III or IV according to Claim 1 and R¹ and R² are hydrogen atoms, A and B are nitrogen atoms and R¹, R³, R⁴, R⁵, R⁶, R⁷, R⁸, R⁹, R¹⁰, R¹¹ and R¹⁶ have the same meaning as above.

4. Compounds according to Claim 1 having one of the general formulae XIII and XIV 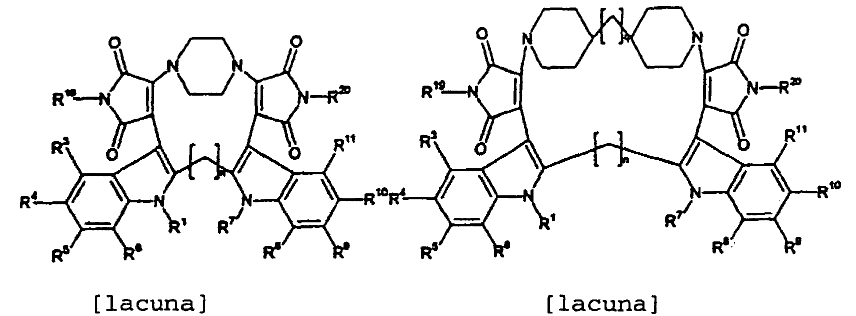 in which n is the numbers 3, 4, 5, 8 or 12, q is the numbers 0, 1, 2, 3, 5 or 6, R¹⁹, R²⁰ are hydrogen atoms or alkyl groups and R¹, R³, R⁴, R⁵, R⁶, R⁷, R⁸, R⁹, R¹⁰, R¹¹ and R¹⁶ are identical or different and have the same meaning as above.

5. Compounds according to Claim 1 having the general formula XV 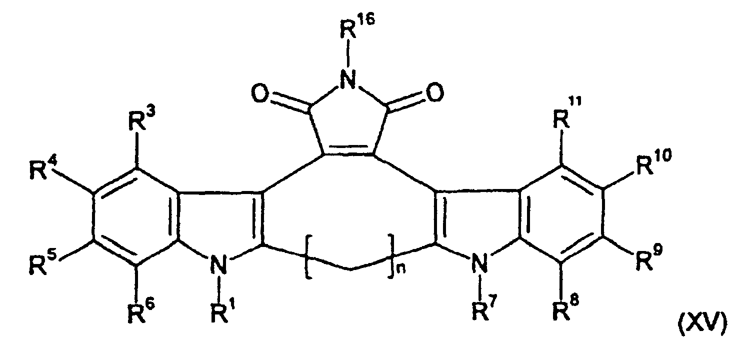 in which n is the numbers 1, 2 or 3, R¹⁶ is a hydrogen atom or an alkyl group and R¹, R³, R⁴, R⁵, R⁶, R⁷, R⁸, R⁹, R¹⁰, R¹¹ and R¹⁶ are identical or different and have the same meaning as above.

6. Bisindol-2-ylmethan-1-one according to Claim 1.

7. (5-Methoxyindol-2-yl)-(indol-2-yl)methan-1-one according to Claim 1.

8. Bis(5-methoxyindol-2-yl)-1-methanone according to Claim 1.

9. Benzo[*b*]thiophen-2-yl(5-methoxy-1*H*-2-indolyl)-1-methanone according to Claim 1.

10. 5-Hydroxy-1*H*-2-indolyl(1H-2-indolyl)methanone according to Claim 1.

11. 1*H*-2-Indolyl[5-(2-morpholin-1-ylethyloxy)-1*H*-2-indolyl]methanone according to Claim 1.

12. 1*H*-2-Indolyl[5-(2-dimethylaminoethyloxy)-1*H*-2-indolyl]methanone according to Claim 1.

13. [2-(1*H*-2-Indolylcarbonyl)-1*H*-5-indolyl) ethanoate according to Claim 1.

14. [2-(1*H*-2-Indolylcarbonyl)-1*H*-5-indolyl) butanoate according to Claim 1.

15. [2-(1*H*-2-Indolylcarbonyl)-1*H*-5-indolyl)] 2-(*N*,*N*)-dimethylaminoethanoate according to Claim 1.

16. [2-(1*H*-2-Indolylcarbonyl)-1*H*-5-indolyl)] propanoate according to Claim 1.

17. [2-(1*H*-2-Indolylcarbonyl)-1*H*-5-indolyl)] 2-thiophenylethanoate according to Claim 1.

18. Medicaments comprising a compound according to one of Claims 1 to 17.

19. Compound of the formula I according to one of Claims 1 to 17 for use as a tyrosine kinase inhibitor.

20. Compound of the formula I according to one of Claims 1 to 17 for use as an inhibitor of a PDGF receptor tyrosine kinase or a structurally related receptor tyrosine kinase.

21. Compound of the formula I according to one of Claims 1 to 17 for use in the treatment of tumours.

22. Compound of the formula I according to one of Claims 1 to 17 for use in the treatment of arteriosclerosis, restenosis after balloon angioplasty, arthritis and fibrotic diseases

23. Process for the preparation of compounds according to Claim 1, in which B is an N atom, R² and R¹³ are a radical having the general formula V according to Claim 1 or together form a linkage having the general formula VII according to Claim 1, **characterized in that** a 2,2'-bis-1*H*-indolylalkane or a derivative thereof having the general formula XI 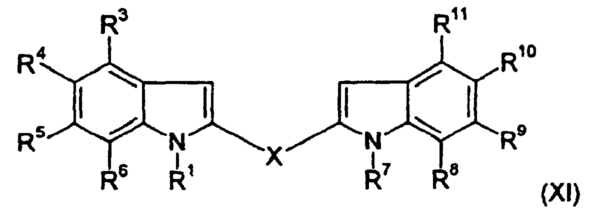 in which X, R¹, R³, R⁴, R⁵, R⁶, R⁷, R⁸, R⁹, R¹⁰, and R¹¹ have the same meaning as above, is reacted with dibromomaleimide.

24. Process for the preparation of compounds according to Claim 1, in which B is an N atom,R² and R¹³ together form a linkage having the general formula IX or X according to Claim 1, **characterized in that** a 2,2'-bis-1*H*-indolylalkane or a derivative thereof having the general formula XI 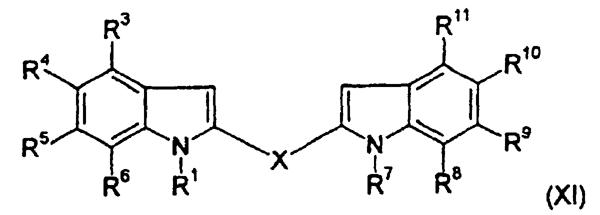 in which X, R¹, R³, R⁴, R⁵, R⁶, R⁷, R⁸, R⁹, R¹⁰ and R¹¹ have the same meaning as above, is initially reacted with dibromomaleimide and then reacted with a primary or secondary amine of the following general structures [sic] XVI, XVII or piperazine 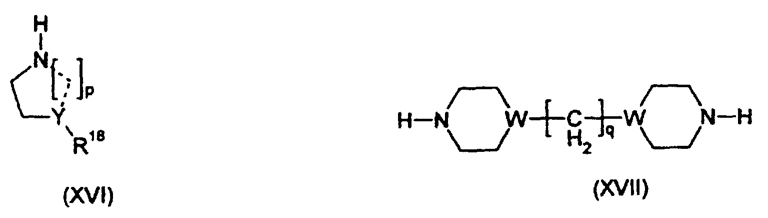 in which p, q, R¹⁷ and W have the same meaning as above.

## Revendications

1. Composés de formule générale I 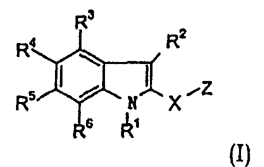 dans laquelle Z est un groupe de formule générale (II) 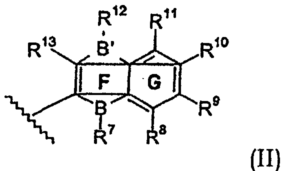 dans laquelle B, B' peuvent être du carbone, un atome d'azote, d'oxygène ou de soufre, et les systèmes cycliques F et G indépendamment l'un de l'autre, peuvent être des cycles de 5 à 6 maillons aussi bien saturés que non saturés, X représente un groupe ayant les formules générales III ou IV,
―(CH₂)ₗ―[CR¹⁴R¹⁵]ₘ―(CH₂)ₙ (III) 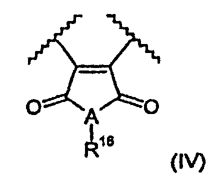
dans laquelle A peut être un atome de N, O ou S, I et n peuvent prendre les nombres de 0 à 6, m les nombres 1 et 2 ainsi que R¹⁴ et R¹⁵ forment soit ensemble un atome d'oxygène, soit R¹⁴ un groupe hydroxyle et R¹⁵ un atome d'hydrogène, ou bien R¹⁴ et R¹⁵ signifient des atomes d'hydrogène, et dans laquelle R¹⁶ signifie un atome d'hydrogène, un reste alkyle ou un reste aryle, un reste alkyle ou aryle substitué par un halogène, un amino ou un azido, un reste alkyloxyméthyle ou un reste alkyloxyméthyle substitué.
R² et R¹³ conjointement forment une liaison ayant les formules générales V ou VI, 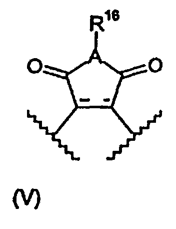 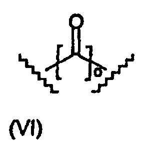 dans lesquelles la liaison en tracé ondulé signifie une liaison double ou simple, A et R¹⁶ possèdent la même signification que ci-dessus et o peut prendre les nombres 1 et 2, ou des atomes d'hydrogène, R² et R¹³ signifient des restes identiques ou différents de formule générale VII 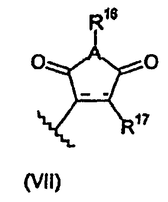 dans laquelle la liaison ondulée signifie une double liaison ou une liaison simple, A et R¹⁶ signifient la même chose que possédé ci-dessus, et R¹⁷ signifie un atome d'halogène ou un ester de la formule générale VIII 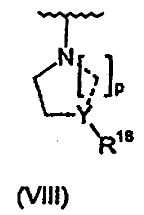 de sorte que p peut être = 0, 1 ou 2 (quand p = 0 alors il s'agit d'une amine primaire acyclique et Y porte un atome d'hydrogène en supplément), Y peut être un carbone, un atome d'oxygène ou d'azote et quand Y est un atome de carbone ou un atome d'azote, R¹⁸ signifie un atome d'hydrogène, ou un reste alkyle ou aryle, un reste alkyle ou aryle substitué, un hétérocycle saturé ou non saturé, un reste alkoxycarbonyle, un reste aminocarbonylméthyle, un reste aminocarbonylméthyle substitué, R² et R¹³ ensemble forment une liaison ayant les formules générales IX ou X, 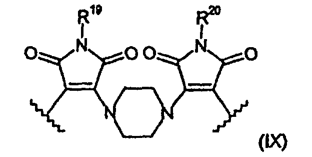 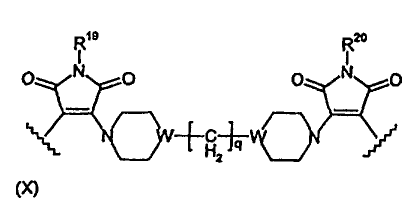 dans lesquelles W représente soit un atome de carbone, soit un atome d'azote, q peut être un nombre compris entre 0 et 6 et R¹⁹ et R²⁰ peuvent signifier des atomes d'hydrogène, des restes alkyle ou alkyle substitués, R¹, R⁷ et R¹² sont identiques ou différents et signifient des restes alkyle et amine alkyle, des restes phénylsulfonyle, des restes alkylsilylméthoxyméthyle, un sucre ou un sucre substitué,
R³, R⁴, R⁵, R⁶, R⁸, R⁹, R¹⁰ et R¹¹ sont identiques ou différents et représentent respectivement un atome d'hydrogène, un groupe alkyl, alkoxy, alkoxyméthyl substitué par un alkoxy, un amino, un halogène, un cycloalkyle, un cyclohétéroalkyle, un aryle ou un hétéroaryle, un groupe nitro, un atome d'halogène, ou un groupe O-alkoxy de formule générale, -O-(C=O)-R²¹ dans laquelle R²¹ signifie un groupe alkyle, alkoxy ou alkoxyméthyle substitué par un alkoxy, un amino, un halogène, un cycloalkyle, un chclohétéroalkyle, un aryle ou un hétéroaryle.

2. Composés selon la revendication 1 ayant la formule générale XI, 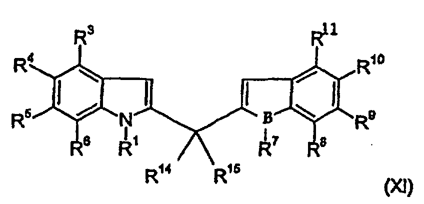 dans laquelle B signifie un atome d'azote, d'oxygène ou de soufre et R³, R⁴, R⁵, R⁶, R⁷, R⁸, R⁹, R¹⁰, R¹¹, R¹⁴ et R¹⁵ ont les mêmes significations que ci-dessus.

3. Composés selon la revendication 1, ayant la formule générale I, dans laquelle X représente un groupe ayant la formule générale III ou IV selon la revendication 1, et R¹ et R² signifient des atomes d'hydrogène, A et B signifient des atomes d'azote ainsi que R¹, R³, R⁴, R⁵, R⁶, R⁷, R⁸, R⁹, R¹⁰, R¹¹, et R¹⁶ ont les mêmes significations que ci-dessus.

4. Composés selon la revendication 1, ayant une des formules générales XIII et XIV, 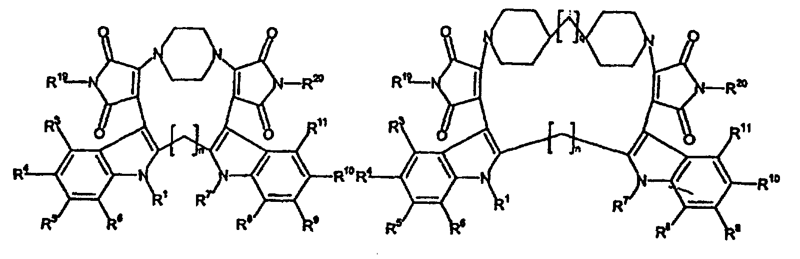 dans lesquelles n signifie des nombres 3, 4, 5, 8 et 12, q signifie les nombres O, 1, 2, 3, 5 et 6, R¹⁹ et R²⁰ signifient de l'hydrogène ou des groupes alkyle ainsi que R¹, R³, R⁴, R⁵, R⁶, R⁷, R⁸, R⁹, R¹⁰, R¹¹, et R¹⁶ sont identiques ou différents, et possèdent les mêmes significations que ci-dessus.

5. Composés selon la revendication 1, ayant la formule générale XV, 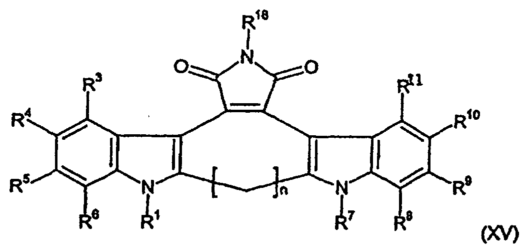 dans laquelle
n signifie les nombres 1, 2, 3, R¹⁶ signifie un atome d'hydrogène ou un groupe alkyle, ainsi que R¹, R³, R⁴, R⁵, R⁶, R⁷, R⁸, R⁹, R1⁰, R¹¹, et R¹⁶ sont identiques ou différents, et possèdent les mêmes significations que ci-dessus.

6. Bisindol-2-ylméthan-1-one selon la revendication 1.

7. (5-méthoxyindol-2-yl)-(indol-2-yl)méthan-1-one selon la revendication 1.

8. Bis(5-méthoxyindol-2-yl)-1-méthanone selon la revendication 1.

9. Benzo[*b*]thiophèn-2-yl(5-méthoxy-1*H*-2-indolyl)-1-méthanone selon la revendication 1.

10. 5-hydroxy-1*H*-2-indolyl(1*H*-2-indolyl)méthanone selon la revendication 1.

11. 1*H*-2-indolyl[5-(2-morpholin-1-yléthyloxy)-1*H*-2-indolyl]méthanone selon la revendication 1.

12. 1*H*-2-indolyl[5-(2-diméthylaminoéthyloxy)-1*H*-2-indolyl]méthanone selon la revendication 1.

13. [2-(1*H*-2-indolylcarbonyl))-1*H*-5-indolyl)]éthanoate selon la revendication 1.

14. [2-(1*H*-2-indolylcarbonyl))-1*H*-5-indolyl)]butanoate selon la revendication 1.

15. [2-(1*H*-2-indolylcarbonyl))-1*H*-5-indolyl]2-(*N*,N)-diméthylaminoéthanoate selon la revendication 1.

16. [2-(1*H*-2-indolylcarbonyl))-1*H*-5-indolyl)]propanoate selon la revendication 1.

17. [2-(1*H*-2-indolylcarbonyl))-1*H*-5-indolyl)]2-thiophényléthanoate selon la revendication 1.

18. Médicaments comprenant un composé selon l'une quelconque des revendications 1 à 17.

19. Composé de formule I selon l'une quelconque des revendications 1 à 17 en vue de l'utilisation comme agent inhibant la tyrosine-kinase.

20. Composé de formule I selon l'une quelconque des revendications 1 à 17 en vue de l'utilisation comme agent inhibant la tyrosine-kinase de récepteur de PDGF ou agent inhibant la tyrosine-kinase de récepteur structurellement apparenté.

21. Composé de formule I selon l'une quelconque des revendications 1 à 17 en vue de l'utilisation des tumeurs.

22. Composé de formule I selon l'une quelconque des revendications 1 à 17 en vue de l'utilisation pour le traitement de l'artériosclérose, de la resténose après angioplaste par ballonnet, l'arthrite et les maladies fibrotiques.

23. Procédé de préparation de composés selon la revendication 1, dans lesquels B signifie un atome de N, R² et R³ un reste ayant la formule générale V selon la revendication 1, ou ensemble forment une liaison ayant la formule générale VII selon la revendication 1, **caractérisé en ce qu'**
on fait réagir un 2,2'-bis-[1H]-indolylalkane ou un dérivé de celui-ci ayant la formule générale XI ? 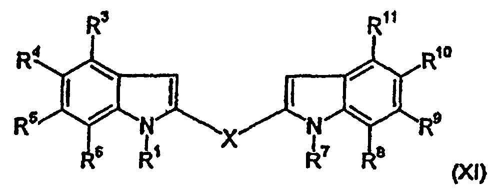 dans laquelle X, R¹, R³, R⁴, R⁵, R⁶, R⁷, R⁸, R⁹, R¹⁰ et R¹¹ possèdent les mêmes significations comme ci-dessus, avec le dibrommaleinimide.

24. Procédé de préparation de composés selon la revendication 1, dans lesquels B forme un atome de N, R² et R¹³ conjointement forment avec liaison ayant la formule générale IX ou X selon la revendication 1,
**caractérisé en ce qu'**
on fait réagir en premier lieu un 2,2'-bis-[1H]-indolylalkane ou un dérivé de celui-ci ayant la formule générale XI, 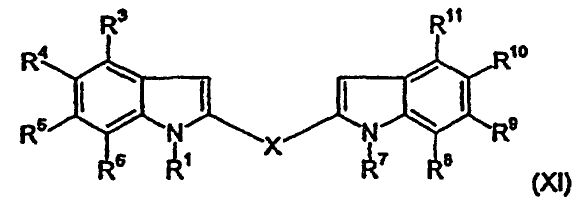 dans laquelle X, R¹ R³, R⁴, R⁵, R⁶, R⁷, R⁸, R⁹, R¹⁰ et R¹¹ possèdent les mêmes significations comme ci-dessus, avec le dibrommaleinimide, et ensuite fait réagir avec une amine primaire ou secondaire de structures générales suivantes XVI, XVII ou avec la pipérazine, 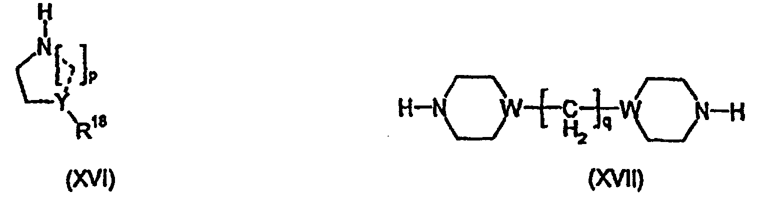 dans lesquelles p, q, R¹⁷ et W possèdent la même signification que ci-dessus.
